# EUROPEAN PATENT APPLICATION

(11) **EP 4 023 289 A1**
(43) Date of publication of application: **06.07.2022**
(21) Application number: 20856779.2
(22) Date of filing: 26.08.2020
(51) Int. Cl.: A61P 1/00, A61P 1/02, A61P 11/02, A61P 15/02, A61P 27/02, A61P 27/16, A61P 43/00, C07H 3/04, A61P 17/02, C12N 5/071, A61K 31/7016

(54) **METHOD FOR PRODUCING CULTURED TISSUE, AND PREPARATION FOR EXTERNAL APPLICATION**

(30) Priority: 29.08.2019 JP 2019156730
(71) Applicant: National University Corporation Ehime University, Matsuyama-shi, Ehime 7908577 (JP)
(72) Inventor: MUTO,Jun, Toon-shi, Ehime 791-0295 (JP); SAYAMA,Koji, Toon-shi, Ehime 791-0295 (JP)
(74) Representative: Cabinet Beau de Loménie
(86) International application number: PCT/JP2020/032153
(87) International publication number: WO 2021/039833

(57) **Abstract**

An object of the present invention is to provide a new means for suppressing inhibition of proliferation of keratinocytes even when the keratinocytes start to come into contact with each other. The present invention solves the above problem by culturing keratinocytes in contact with a common medium with fibroblasts treated with a composition containing a specific compound.

## Description

### TECHNICAL FIELD

The present invention relates to a method for producing a cultured tissue, a cultured tissue obtained by the method, a preparation for external application, and a composition for fibroblast activation and the like.

### BACKGROUND ART

A human three-dimensional skin model having an epithelial tissue layer on a dermal tissue layer is used for a skin sample for in vitro test as a model that reproduces or mimics structure of human skin, particularly structure of a portion including epidermis and dermis and its environment, in research and development of a composition for external application.

In order to obtain such a three-dimensional cultured tissue including an epithelial tissue layer and a dermal tissue layer, means are known in which an amnion from which an epithelium is removed is disposed on a collagen matrix in which fibroblasts are embedded, and normal human keratinocytes are induced to differentiate thereon to form an epithelial tissue layer (for example, Patent Document 1, Non-Patent Document 1, and the like). Furthermore, a means is known for preparing a dense dermis-like tissue using a specific reactor circulating a cell culture fluid containing extracellular matrix components and cells, and then forming an epithelial tissue layer using recombinant proteins and epithelial cells (for example, Patent Document 2).

On the other hand, a therapeutic method for promoting recovery of wounds of skin such as burns by transplanting artificially cultured skin epithelium has already been put into practical use. In addition, a research using a sheet of corneal epithelium obtained by culturing corneal epithelial cells for transplantation is in progress. These cultured epithelia such as skin epithelium and corneal epithelium are obtained by culturing keratinocytes, which are epithelial cells, until they are multilayered and cornified to form an epithelial tissue layer.

According to Non-Patent Document 2, as a method for obtaining only the epithelial tissue layer, a means of peeling the epithelial tissue layer from the three-dimensional cultured tissue by treatment with dispase or the like to be formed into a sheet is known. In addition, a means for three-dimensionally culturing keratinocytes on a collagen gel (for example, Patent Document 3), a Green's method for obtaining an epithelial tissue layer by culturing keratinocytes in a common medium with 3T3 cells as feeder cells and the like are known.

### PRIOR ART DOCUMENTS

### PATENT DOCUMENT

Patent Document 1: WO 2005/087286 A
Patent Document 2: JP-A-2010-172247
Patent Document 3: JP-B2-2773058

### NON-PATENT DOCUMENT

Non-Patent Document 1: Hashimoto et al., Cell Tissue Res . 2006, 69, 326.
Non-Patent Document 2 : Inoue. et al., Organ Biology (Jpn), 2015, Vol.22, No.1, p.57-65.

### SUMMARY OF THE INVENTION

### PROBLEMS TO BE SOLVED BY THE INVENTION

However, in the case of forming an epithelial tissue layer on a three-dimensional cultured tissue, there is a problem that cell proliferation is suppressed by so-called contact inhibition when keratinocytes are densely packed and start to contact by culture. As a result, an area of the epithelial tissue layer does not enlarge. On the other hand, in order to efficiently proliferate keratinocytes, it is necessary to culture cells at a certain high density. Therefore, in order to obtain a large area of cultured epithelium, keratinocytes must be cultured in a large amount in advance and then seeded at a high density on the dermal tissue layer. As a result, it takes a long period of time to prepare the cultured epithelium.

Further, as in Patent Document 1 and Non-Patent Document 1, when a collagen gel is used as a support, contraction of the collagen gel occurs, and thickness unevenness, wrinkles and twists of the epithelial tissue layer and the like may occur. Furthermore, in the method of Patent Document 2, a special device is required for formation of a dermal tissue layer, and a recombinant protein combining a cell growth factor and a collagen-binding domain is required for formation of an epithelial tissue layer.

On the other hand, when a special feeder cell is used as in the Green's method, there is a possibility that an antigen derived from the feeder cell or the like is mixed. In particular, in the case of obtaining a cultured epithelium for autologous epithelial transplantation, a means for antigen removal is required. In addition, this method cannot be used as a method for obtaining a three-dimensional cultured tissue.

Therefore, an object of the present invention is to provide a new means for suppressing inhibition of proliferation of keratinocytes even when the keratinocytes start to come into contact with each other.

Specifically, the present invention relates to providing a new means of in vitro culture capable of enlarging the area of the epithelial tissue layer.

Also, specifically, the present invention relates to providing a new composition for external application that promotes formation of an epithelial tissue layer in a wound part of a living body.

### MEANS FOR SOLVING THE PROBLEMS

As a result of intensive studies, the present inventors have found that when keratinocytes are cultured in contact with a common medium with fibroblasts treated with a composition containing a specific compound, even when keratinocytes start to come into contact with each other, proliferation of the keratinocytes is suppressed, and as a result, the area of the epithelial tissue layer continuously increases by culture, and have completed the present invention.

That is, the present invention provides the following method for producing a cultured tissue having an epithelial tissue layer.
[1] A method for producing a cultured tissue having an epithelial tissue layer, the production method comprising:
   a step of culturing keratinocytes in contact with a common medium with fibroblasts treated with a composition containing trehalose or a derivative thereof to form an epithelial tissue layer.
[2] The production method according to [1], wherein
   the fibroblasts are included in a cell support to constitute a dermal cell structure, and
   the dermal cell structure is a layered structure, and the keratinocytes are present on the layered structure of the dermal cell structure.
[3] The production method according to [1] or [2], further comprising a step of separating the epithelial tissue layer,
   wherein the cultured tissue having an epithelial tissue layer is a cultured epithelium.
[4] The production method according to any one of [1] to [3], wherein a total content of trehalose and a derivative thereof in the composition containing trehalose or a derivative thereof is 10 to 300 mg/mL in terms of trehalose.
   The present invention also provides the following cultured tissue.
[5] A cultured tissue comprising keratinocytes cultured in contact with a common medium together with a dermal cell structure,
   wherein the dermal cell structure comprises trehalose or a derivative thereof, a cell support, and fibroblasts,
   wherein the keratinocytes contain cornified keratinocytes.
[6] A cultured tissue having a dermal tissue layer comprising at least fibroblasts, wherein the proportion of the fibroblasts in G2 phase of cell cycle to the total number of the fibroblasts is 1 to 50%.
   Furthermore, the present inventors have found that the specific compound promotes formation of an epithelial tissue layer in a wound part of a living body, and have completed the invention of the following preparation for external application.
[7] A preparation for external application for treatment of an epithelial wound, comprising trehalose or a derivative thereof as an active ingredient.
[8] The preparation for external application according to [7], wherein a total content of trehalose and a derivative thereof is 5 to 30% by mass in terms of trehalose.
[9] The preparation for external application according to [7] or [8], which is a skin preparation, an ophthalmic preparation, an oral preparation, an otic preparation, a nasal preparation, a rectal preparation, or a vaginal preparation.
   Furthermore, the present invention provides the following composition for ERK1/2 activation, AKT activation, FGF2 expression promotion, cell activation, or cell proliferation.
[10] A composition containing trehalose or a derivative thereof, which is applied to fibroblasts, and is used for ERK1/2 activation, AKT activation, FGF2 expression promotion, cell activation, or cell proliferation.

### EFFECT OF THE INVENTION

According to the method for producing a cultured tissue of the present invention, even when keratinocytes start to come into contact with each other, inhibition of proliferation of the keratinocytes is suppressed, so that it is possible to continuously enlarge the area of the epithelial tissue layer by continuously culturing the epithelial tissue layer.

As a result, the step of proliferating keratinocytes can be performed simultaneously with the step of forming an epithelial tissue layer including multilayering and cornification, and the step of proliferating keratinocytes in advance can be omitted, so that the culture time for obtaining a cultured tissue can be greatly shortened.

The preparation for external application of the present invention can promote formation of epithelium in a wound part of a living body and improve the wound state.

### BRIEF DESCRIPTION OF THE DRAWINGS

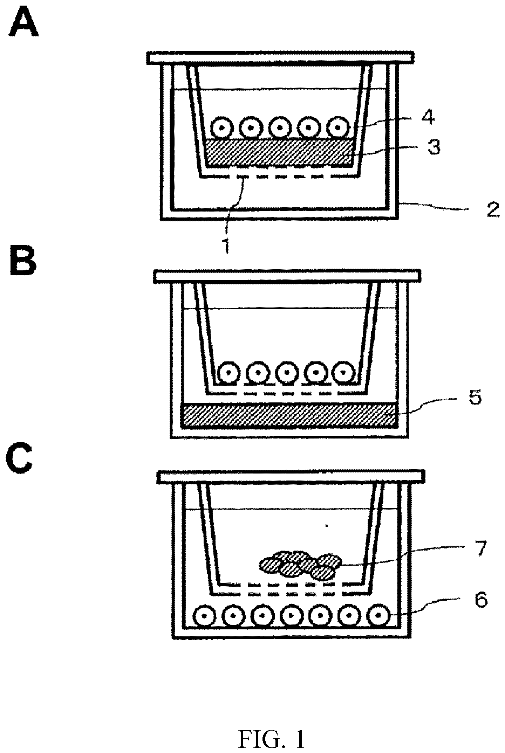

Fig. 1 shows schematic views showing an example of a specific embodiment of a method for producing a cultured tissue. (A) is a schematic view showing an example in which keratinocytes are present on a layered dermal cell structure. (B) is a schematic view showing an example in which keratinocytes exist on a plane, and a dermal cell structure and the keratinocytes are cultured in a separated state. The keratinocytes are present in a plane on a cell culture insert, and the dermal cell structure forms a layered dermal tissue layer at a bottom surface of a cell culture dish. (C) is a schematic view showing an example in which keratinocytes exist on a plane, and a dermal cell structure and the keratinocytes are cultured in a separated state. The keratinocytes are present on a bottom surface of a cell culture dish.

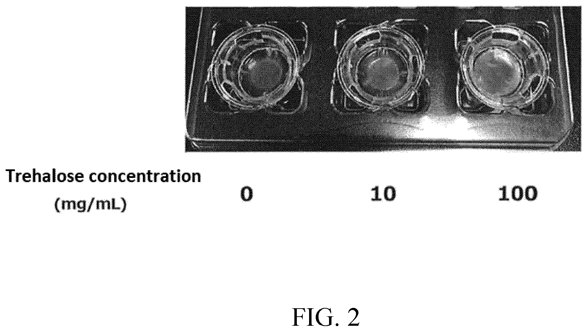

Fig. 2 is a photograph comparing areas of epithelial tissue layers obtained by culturing keratinocytes on a dermal tissue layer containing 0, 10, or 100 mg/mL trehalose in Test Example 1.

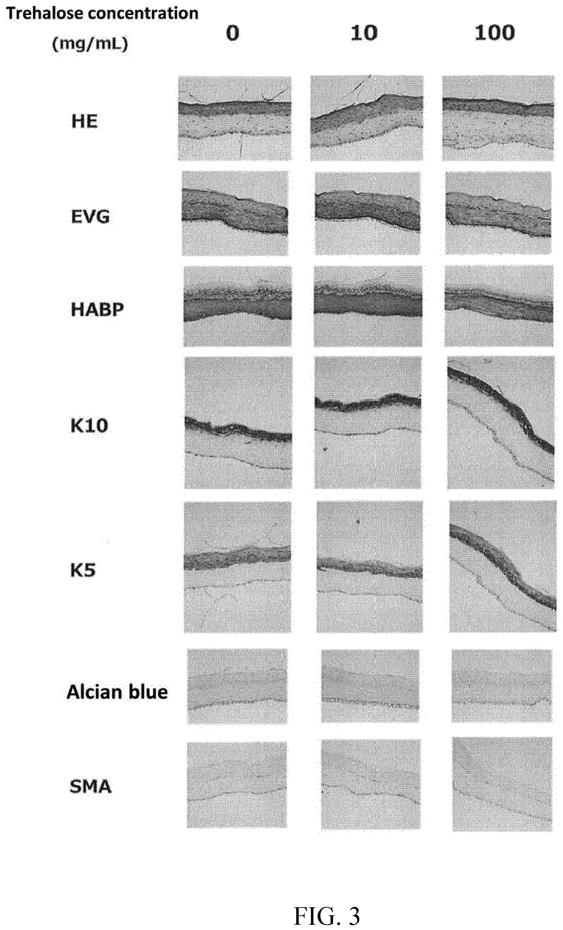

Fig. 3 shows stained images of a transverse section of a three-dimensional cultured tissue obtained by culturing on a dermal tissue layer containing 0, 10, or 100 mg/mL trehalose in Test Example 1.

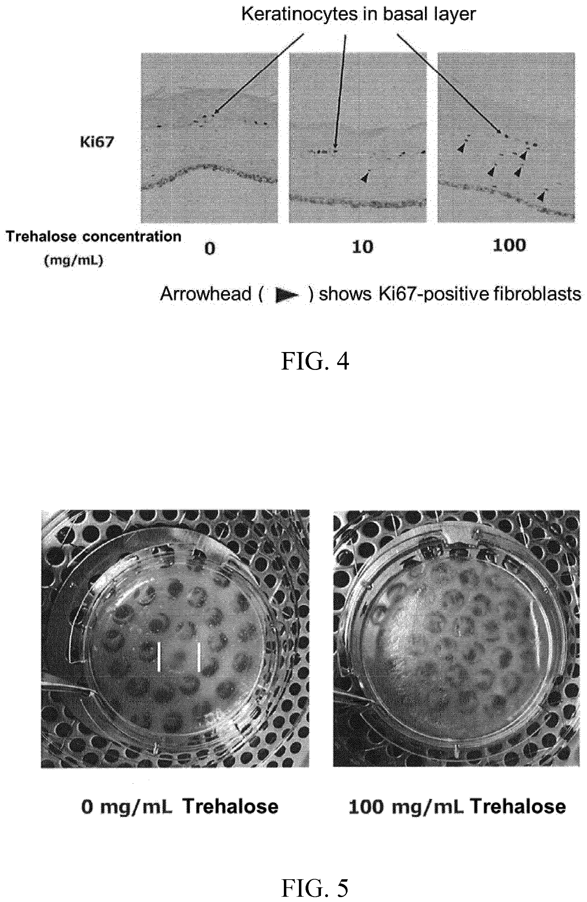

Fig. 4 shows Ki67 stained images of a transverse section of a three-dimensional cultured tissue obtained by culturing on a dermal tissue layer containing 0, 10, or 100 mg/mL trehalose in Test Example 1. Arrowheads indicate portions of Ki67 positive fibroblasts. Note that, in a basal layer near a boundary between an epithelial tissue layer and the dermal tissue layer, there are keratinocytes positive for Ki67.
Fig. 5 shows photographs comparing areas of epithelial tissue layers after gas-liquid interface culture of a sheet with a diameter of about 1 cm composed of confluent keratinocytes on a dermal tissue layer containing 0 or 100 mg/mL trehalose in Test Example 2. In the left photograph (0 mg/mL trehalose treatment), a width between white lines corresponds to a diameter of the obtained epithelial tissue layer. In the right photograph (100 mg/mL trehalose treatment), cells form a sheet over an entire filter insert.

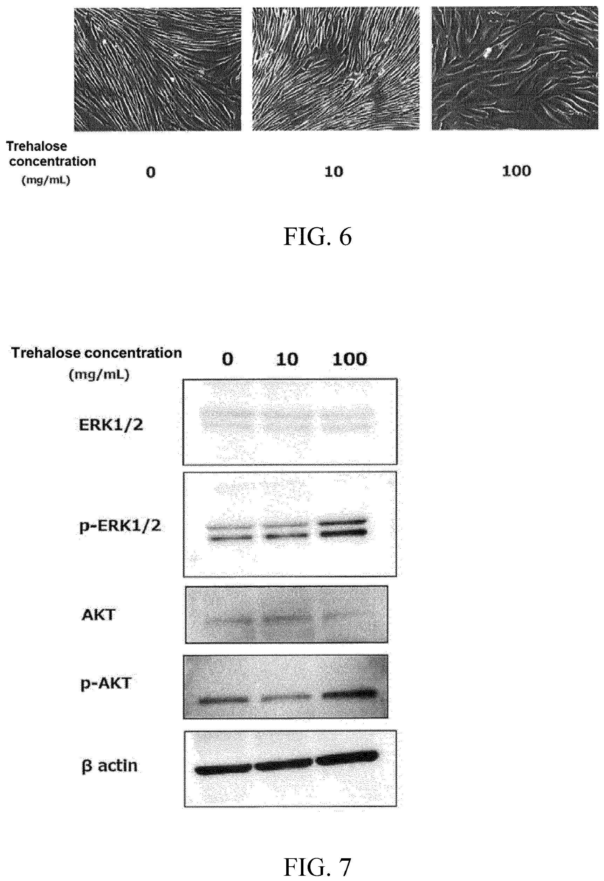

Fig. 6 shows optical microscopic images showing cell morphology after culture of human normal fibroblasts cultured in a monolayer in a medium containing 0, 10 or 100 mg/mL trehalose in Test Example 3-1.
Fig. 7 shows Western blots of various proteins extracted from human normal fibroblasts cultured in monolayers in a medium containing 0, 10 or 100 mg/mL trehalose in Test Example 3-1. p-ERK and p-AKT represent Thr202/Tyr204 phosphorylated ERK1/2 and Ser473 phosphorylated AKT, respectively.

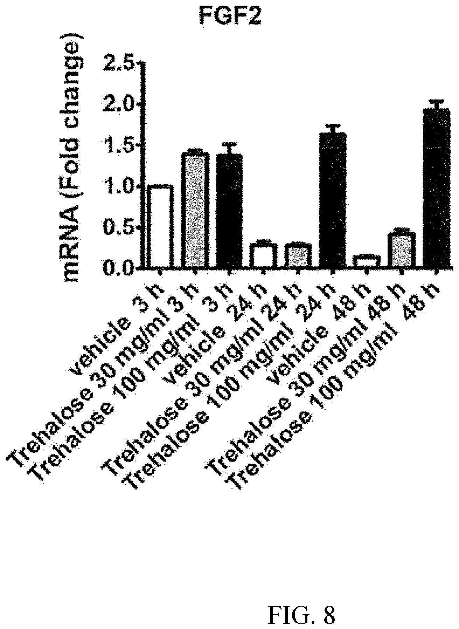

Fig. 8 is a graph comparing FGF2 mRNA amounts in human normal fibroblasts cultured in a monolayer for 3, 24 or 48 hours in a medium containing 0, 30 or 100 mg/mL trehalose in Test Example 3-2. The vertical axis represents mRNA amount relativized using mRNA amount when cultured at a trehalose concentration of 0 mg/mL (vehicle) for 3 hours as 1. The number of samples is 6, and error bars represent standard errors.

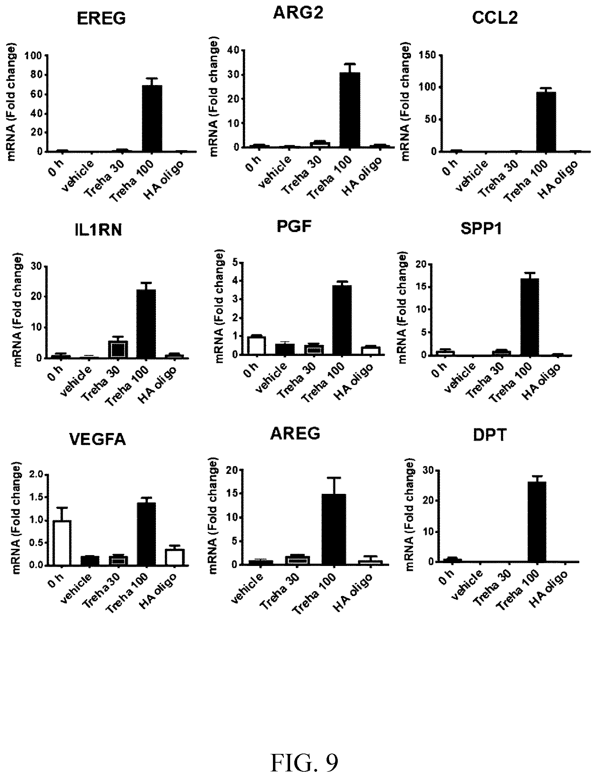

Fig. 9 shows graphs comparing mRNA amounts of various genes of human normal fibroblasts cultured for 24 hours under various medium conditions in Test Example 3-3. The vertical axis represents mRNA amount relativized using mRNA amount of human normal fibroblasts before trehalose treatment (0h) as 1. The number of samples is 3, and error bars represent standard errors.

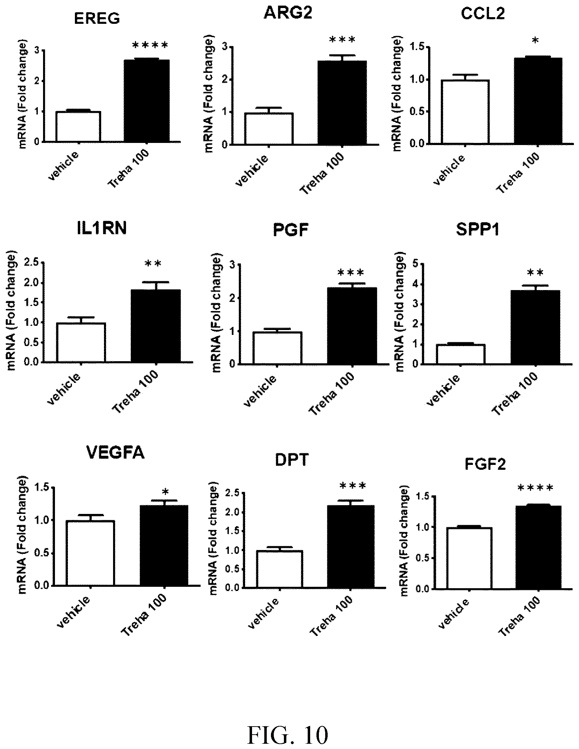

Fig. 10 shows graphs comparing mRNA amounts of various genes of human normal fibroblasts cultured for 3 days under various medium conditions in Test Example 3-4. The vertical axis represents mRNA amount relativized using mRNA amount of human normal fibroblasts cultured in a medium not containing trehalose (vehicle) as 1. The number of samples is 3, and error bars represent standard errors. * is p < 0.05, ** is p < 0.01, *** is p < 0.001, and **** is p < 0.0001 (all are unpaired t-tests) .

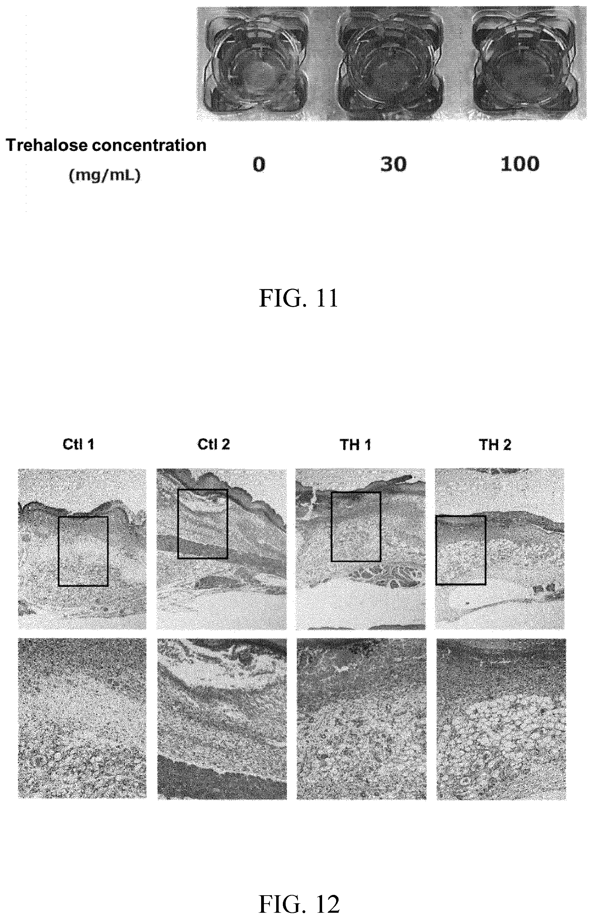

Fig. 11 shows photographs comparing areas of epithelial tissue layers obtained by culturing keratinocytes on a dermal tissue layer containing fibroblasts precultured in a medium containing 0, 30, or 100 mg/mL trehalose in Test Example 4.
Fig. 12 shows HE stained images of biopsy samples of transplantation parts 5 days after transplantation of control groups (Ctl1, 2) and trehalose groups (TH1, 2) in Test Example 5. A lower image of each group is an image obtained by enlarging an image within a black frame of an upper image.
Fig. 13 shows a change in area of a wound formed on the back of a mouse in Test Example 6. (A) is a graph showing a change in wound area with respect to the number of days after the start of the test. Wound is formed on day 1. The vertical axis represents a relative value when wound area immediately after wound formation is 100%. Two-way ANOVA showed significant differences between groups in wound area at each point of days 2 to 6 (n = 6 in each group, p < 0.001). Error bars represent standard errors. (B) shows photographs of wound parts of trehalose treatment group and control group taken at 0 hour, 24 hours, 48 hours, and 72 hours after wound formation, as diagrams showing representative changes in wounds.

### MODE FOR CARRYING OUT THE INVENTION

### [Definitions]

In the present specification, the "dermal cell structure" refers to a structure containing at least fibroblasts as main cells, and a cell support, in which the fibroblasts exist two dimensionally or three dimensionally, and the cell support is included in the gap. Specific examples of the fibroblasts include skin fibroblasts and corneal parenchymal cells.

The dermal cell structure preferably resembles or simulates morphology of dermis such as skin or cornea. The dermal cell structure may further contain a tissue appendage and/or cells constituting the tissue appendage. Examples of the tissue appendage include blood vessels, lymphatic vessels, sebaceous glands, sweat glands, hairs, hair follicles, and the like. Examples of the cells constituting the tissue appendage include vascular endothelial cells, lymphatic endothelial cells, immune cells, melanocytes, dendritic cells, Langerhans cells, hair follicle cells, hair papilla cells, sebaceous gland cells, adipocytes, and the like.

Also, the dermal cell structure may be the dermis itself such as skin or cornea from an organism. In this case, the cell support is an extracellular matrix.

In the present specification, the "dermal tissue layer" refers to a layered dermal cell structure.

In the present specification, the "cell support" refers to a substance that fills a space outside the cell, and has a skeletal role, a function of providing a scaffold, a function of retaining a biological substance such as a growth factor, and the like. The cell support includes, for example, a biological substance such as an extracellular matrix, a culture support such as collagen gel used for three-dimensional culture, and the like.

In the present specification, the "epithelial cell layer" is a layered structure containing keratinocytes as main cells, and refers to a structure composed of keratinocytes that are not multilayered or cornified. The epithelial cell layer may further contain stem cells of keratinocytes, basal cells, spinous cells, or the like.

In the present specification, the "epithelial tissue layer" is a two-dimensional or three-dimensional layered substance containing keratinocytes as main cells, and refers to a structure containing multilayered and/or cornified keratinocytes. The epithelial tissue layer may further contain stem cells of keratinocytes, basal cells, spinous cells, or the like.

In the present specification, the "three-dimensional cultured tissue" refers to a cellular tissue in which cells exist three dimensionally, which is obtained by culture.

In the present specification, the "cultured epithelium" refers to an epithelial tissue layer obtained by culture and substantially free of a dermal tissue layer.

In the present specification, the "trehalose" is a disaccharide in which two **α**-glucose are 1,1-glycosidically linked, and is also called **α**,**α**-trehalose. The trehalose herein includes hydrates such as anhydrides and dihydrates.

In the present specification, the "trehalose derivative" refers to a carbohydrate derivative of trehalose, and is a carbohydrate in which one or more sugar monomers are condensed in an **α**,**α**-trehalose structure in a molecule. Specific examples of the trehalose derivative include, but are not particularly limited to, carbohydrate derivatives of **α**,**α'**-trehalose having a degree of glucose polymerization of 3 to 6, such as mono-glucosyl **α**,**α**-trehalose such as α-maltosyl **α**-glucoside and **α**-isomaltosyl **α**-glucoside; di-glucosyl **α**,**α**-trehalose such as **α**-maltotriosyl **α**-glucoside (also known as **α**-maltosyl **α**,**α**-trehalose), **α**-maltosyl **α**-maltoside, **α**-isomaltosyl **α**-maltoside, and **α**-isomaltosyl **α**-isomaltoside; tri-glucosyl **α**,**α**-trehalose such as **α**-maltotetraosyl **α**-glucoside (also known as **α**-maltotriosyl **α**,**α**-trehalose) , **α**-maltosyl **α**-maltotrioside, and **α**-panosyl **α**-maltoside; tetra-glucosyl **α**,**α**-trehalose such as **α**-maltopentaosyl **α**-glucoside (also known as **α**-maltotetraosyl **α**,**α**-trehalose), **α**-maltotriosyl **α**-maltotrioside, and **α**-panosyl **α**-maltotrioside, described in, for example, JP-A-7-143876, JP-A-8-73504, JP-B2-3182679, JP-A-2000-228980, and the like.

The origin and production method of trehalose or a derivative thereof are not particularly limited, and trehalose or a derivative thereof may be derived from a natural product or a chemically synthesized product, but it is preferable that the content of lipopolysaccharide (LPS) or other substances that cause cytotoxicity is an amount that does not interfere with cell proliferation or less.

In one embodiment, the trehalose derivative may release trehalose extracellularly or intracellularly, for example, by metabolism.

In the present specification, the amount in terms of trehalose refers to a mass corresponding to a trehalose moiety obtained by subtracting a sugar moiety other than hydration water and trehalose from trehalose and a derivative thereof.

In the present specification, the "wound" represents an injury suffered by a subject and is a condition in which at least epithelial tissue is damaged. Wounds also include disruption of dermis. Examples of cause of the wound include an incision wound, a chop wound, a stab wound, a lacerated wound, a contused wound, an impalement injury, an operation, a burn, damage to a lower tissue due to decubitus or blood circulation failure, and the like. In the present specification, the wounds include both acute wounds and chronic wounds.

In the present specification, the "improvement" refers to curing, recovering or alleviating a disease, a symptom, a health condition or an aesthetic condition, or preventing or delaying progression of deterioration of a disease, a symptom, a health condition or an aesthetic condition.

In the present specification, unit "% by mass" of a content is synonymous with "g/100 g".

In the present specification, "v/v%" represents a volume percentage and is synonymous with "mL/100 mL".

Abbreviations of gene names herein are notations used in the official symbols of NCBI Gene Database (https://www.ncbi.nlm.nih.gov/gene) .

### [Method for Producing Cultured Tissue]

The method for producing a cultured tissue according to an embodiment of the present invention includes a step of culturing keratinocytes in contact with a common medium with fibroblasts treated with a composition containing trehalose or a derivative thereof to form an epithelial tissue layer.

The cultured tissue obtained by the production method of the present invention includes an epithelial tissue layer. Examples of such a cultured tissue include a cultured epithelium, a three-dimensional cultured tissue including at least an epithelial tissue layer and a dermal tissue layer, and the like.

### (Fibroblasts treated with composition containing trehalose or derivative thereof)

In the method for producing a cultured tissue of the present invention, an aspect of localization of fibroblasts treated with a composition containing trehalose or a derivative thereof is not particularly limited as long as the fibroblasts are in contact with a common medium with keratinocytes and as long as the fibroblasts do not interfere with formation of an epithelial tissue layer from keratinocytes. For example, as described later, the fibroblasts may be present in a common medium as, for example, a dermal cell structure having a layered or massive form, or may exist as a single layer of adherent cells . When a three-dimensional cultured tissue including an epithelial tissue layer and a dermal tissue layer is prepared, it is preferable that fibroblasts are included in a cell support to constitute a layered dermal cell structure.

Treating fibroblasts with a composition containing trehalose or a derivative thereof refers to incubating fibroblasts in contact with a composition such as a solution containing trehalose or a derivative thereof or a cell support for a specific period of time.

Fibroblasts to be treated are not particularly limited, but normal fibroblasts are preferred. Such fibroblasts are not particularly limited, and examples thereof include fibroblasts collected from skin, cornea or the like from a healthy subject, or skin, cornea or the like from a subject in need of wound treatment, or cultured cells thereof. In addition, fibroblasts obtained by differentiating from stem cells such as dermal stem cells, mesenchymal stem cells and induced pluripotent stem cells (iPS cells) may be used. The fibroblast-derived animal is not particularly limited, and is preferably a mammal, and more preferably a human. In addition, the fibroblast-derived tissue is preferably skin or cornea, and more preferably skin.

When the fibroblasts are treated with a composition containing trehalose or a derivative thereof, localization of the fibroblasts in the composition is not particularly limited, and the fibroblasts may exist randomly, two dimensionally or three dimensionally, or in a single layer or a plurality of layers.

The form of the composition containing trehalose or a derivative thereof is preferably a solution or a cell support.

The solution is not particularly limited as long as it does not inhibit proliferation of fibroblasts, and examples thereof include isotonic solutions such as phosphate buffered saline (PBS), various cell media, and the like, and a fibroblast medium is preferred. Specifically, the fibroblast medium is preferably a medium obtained by adding a serum such as fetal bovine serum (FCS) to a Dulbecco's Modified Eagle medium (DMEM or DME). The addition amount of FCS is 1 to 20 v/v% and preferably 1 to 10 v/v% with respect to the total amount of the medium. The fibroblast medium may further contain ascorbic acid, antibiotics, growth factors, and the like.

Examples of the cell support used in the production method of the present invention include collagen, gelatin, hyaluronic acid, fibronectin, laminin, fibrin, and extracellular matrix mixtures (for example, Matrigel (registered trademark, manufactured by Corning) and the like). The cell support preferably contains any one or two or more of these cell supports, and more preferably contains at least collagen. The cell support may be in the form of a gel, a sponge, a network, or the like. The cell support is preferably a collagen gel or a collagen sponge, and more preferably a collagen gel, in that the fibroblast can be well cultured.

When the fibroblasts exist two dimensionally (for example, monolayer culture), the density of fibroblasts in the above treatment is not particularly limited as long as the fibroblasts do not reach confluence, and is, for example, 3 × 10³ to × 1 × 10⁵ cells/cm², and preferably 5 × 10³ to 8 × 10⁴ cells/cm². Also, when the fibroblasts exist three dimensionally, the density of fibroblasts is, for example, 5 × 10³ to 8 × 10⁵ cells/cm³, preferably 1 × 10⁴ to 4 × 10⁵ cells/cm³, and more preferably 2 × 10⁴ to 2 × 10⁵ cells/cm³.

The total content of trehalose and a derivative thereof in the composition used for the above treatment with respect to the total amount of the composition is 10 mg/mL or more, preferably 20 mg/mL or more, more preferably 30 mg/mL or more, and further preferably 50 mg/mL or more, in terms of trehalose, from the viewpoint of remarkably exhibiting the effect of the present invention.

The total content of trehalose and a derivative thereof in the composition used for the above treatment with respect to the total amount of the composition is 300 mg/mL or less, preferably 200 mg/mL or less, more preferably 150 mg/mL or less, and further preferably 100 mg/mL or less, in terms of trehalose.

The incubation temperature when performing the above treatment is, for example, 4 to 42°C, preferably 20 to 40°C, more preferably 30 to 37°C, and further preferably 37°C.

The period of the treatment is, for example, 2 hours or more, preferably 5 hours or more, further preferably 12 hours or more, and particularly preferably 24 hours or more at 37°C, from the viewpoint of remarkably exhibiting the effect of the present invention.

The period of the treatment may be, for example, 10 days or less, 7 days or less, 5 days or less, 3 days or less, or 2 days or less at 37°C.

In the method for producing a cultured tissue of the present invention, the density of fibroblasts cultured in a medium common to keratinocytes is, for example, 5 × 10³ to 8 × 10⁵ cells/cm³, preferably 1 × 10⁴ to 4 × 10⁵ cells/cm³, and more preferably 2 × 10⁴ to 2 × 10⁵ cells/cm³.

### (Keratinocytes)

In the method for producing a cultured tissue of the present invention, keratinocytes exist on a plane on which keratinocytes can proliferate, and are cultured in contact with a common medium with the fibroblasts treated with trehalose or a derivative thereof.

In the present specification, a state in contact with a common medium refers to a state in which substances released from fibroblasts treated with trehalose or a derivative thereof can reach keratinocytes through the medium. That is, for example, even when fibroblasts and keratinocytes are separated by a porous body, they are considered to be in contact with a common medium as long as the medium can pass through pores.

Keratinocytes may be present as individual cells spaced apart on the plane on which keratinocytes can proliferate, or may be present as a cell assembly, for example, like an epithelial tissue layer. In addition, the keratinocytes may be present entirely or locally in the plane. Moreover, according to the configuration of the present invention, even when keratinocytes are localized in a part, suppression of expansion of the epithelial tissue layer due to contact inhibition does not occur. Therefore, in the production method of the present invention, for example, instead of seeding keratinocytes, the epithelial tissue layer may be placed on a plane and cultured.

The keratinocytes are not particularly limited, and examples thereof include keratinocytes collected from the skin, cornea or the like of a healthy subject or a patient with a hereditary disease, or the skin, cornea or the like of a subject in need of wound treatment, or cultured cells thereof. In addition, keratinocytes obtained by differentiating from stem cells such as epidermal stem cells and induced pluripotent stem cells (iPS cells) derived from a healthy subject or a patient with a hereditary disease may be used. From establishment of these stem cells to differentiation induction may optionally involve editing or recombination of DNA of the cells.

Among them, the keratinocytes used in the method for producing a cultured tissue of the present invention are not particularly limited, but normal keratinocytes are preferred. The animal from which keratinocytes are derived is not particularly limited, a mammal is preferred, and human is more preferred. Also, the tissue from which keratinocytes are derived is usually the same as the tissue from which fibroblasts are derived, and is preferably skin or cornea, and more preferably skin.

In the case of seeding keratinocytes, the cell density of keratinocytes is not particularly limited, but from the viewpoint of promoting proliferation of the keratinocytes, it is preferable that the cell density is, for example, 2 × 10³ to 3.6 × 10⁵ cells/cm², preferably 4.5 × 10³ to 1.8 × 10⁵ cells/cm², and more preferably 9 × 10³ to 9 × 10⁴ cells/cm² in the whole or a part of the plane capable of culturing cells.

The fibroblasts and the keratinocytes used in the method for producing a cultured tissue of the present invention are preferably derived from the same species as the subject to be transplanted, more preferably derived from the same species and the same individual as the subject to be transplanted, and further preferably derived from the subject to be transplanted, from the viewpoint of reducing rejection at the time of transplantation of cultured tissue to be obtained.

The plane on which the keratinocytes are present is not particularly limited as long as the keratinocytes can adhere and proliferate during culture, and the epithelial tissue layer obtained by culture is formed into sheet. Specific examples of the object constituting such a plane include, in addition to a surface of a dermal tissue layer, a surface of culture tool such as a bottom surface of a cell culture dish, or a porous body such as a cell culture insert or a filter membrane.

When a surface of culture tool is used as the plane, it is preferable that the culture tool is a porous body capable of passing the medium, from the viewpoint of supply of the medium and ease of gas-liquid interface culture described later. Furthermore, the surface of the culture tool is not particularly limited, but for example, it is preferable that the surface is coated with a temperature-responsive polymer so that keratinocytes can be easily separated from the plane by changing the temperature. Specific examples of the temperature-responsive polymer are not particularly limited, and examples thereof include an acrylic polymer or a methacrylic polymer. More specific examples thereof include poly-N-isopropylacrylamide (PNIPAAm), poly-N-n-propylacrylamide, poly-N-n-propylmethacrylamide, poly-N-ethoxyethylacrylamide, poly-N-tetrahydrofurfurylacrylamide, poly-N-tetrahydrofurfurylmethacrylamide, poly-N,N-diethylacrylamide, and the like.

### (Common medium, culture conditions, culture tools)

By culturing the fibroblasts treated with a composition containing trehalose or a derivative thereof and keratinocytes in a common medium, an epithelial cell layer is first formed from the keratinocytes, and then an epithelial tissue layer is formed.

The step of culturing the fibroblasts and keratinocytes in a common medium can be performed until an epithelial tissue layer in which multilayering and cornification are completely advanced is formed. In addition, after performing up to a stage where cornification and multilayering of keratinocytes start to occur, that is, a stage where an epithelial tissue layer starts to be formed from an epithelial cell layer, only an epithelial tissue layer portion including keratinocytes may be cultured to promote multilayering and cornification.

The culture temperature for forming an epithelial cell layer may be appropriately changed, and is, for example, 4 to 42°C, preferably 20 to 40°C, and more preferably 30 to 37°C. In addition, the culture time is, for example, 12 hours to 7 days, preferably 1 to 5 days, more preferably 2 to 3 days, and further preferably 2 days.

The medium for forming an epithelial cell layer is not particularly limited, and can be appropriately selected according to the cell, and a medium used for proliferation of keratinocytes is preferred. Examples of the medium used for proliferation of keratinocytes include a serum-free medium. Examples of the serum-free medium include MCDB153 medium, EpiLife (registered trademark) medium, a medium in which amino acid composition or the like of these media has been modified, a medium in which DMEM and Ham's F-12 media have been mixed at a predetermined ratio, or the like. Examples of the medium in which amino acid composition or the like of the MCDB153 medium has been modified include Growth Medium 2 of Example 2 of JP-B2-3066624 (referred to as MCDB153 Type-II medium in the present specification). The medium for forming an epithelial cell layer used in the production method of the present invention is preferably a medium obtained by mixing MCDB153 Type-II medium, DMEM medium and Ham's F-12 medium at a ratio of 4 : 3 : 1.

It is preferable that one or two or more selected from the group consisting of ethanolamine, O-phosphoethanolamine, hydrocortisone, insulin, adenine, transferrin, selenous acid, triiodothyronine, choline chloride, serine, linoleic acid/BSA, calcium chloride, ascorbic acid and progesterone are further added to the medium for forming an epithelial cell layer, from the viewpoint of promoting growth of cells.

The medium for forming an epithelial cell layer may further contain, for example, salts, vitamins, or the like. Examples of the salts include potassium chloride, sodium chloride, magnesium chloride, disodium monohydrogen phosphate, and the like. Examples of the vitamins include cyanocobalamin, nicotinic acid amide, D-pantothenic acid or a salt thereof, pyridoxine hydrochloride or pyridoxal hydrochloride, D-biotin, thiamine hydrochloride, riboflavin, folic acid, DL-α-lipoic acid, myo-inositol, and the like. The medium for forming an epithelial cell layer can contain one or two or more selected from the group consisting of these salts and vitamins.

The culture for forming an epithelial cell layer is preferably performed on a porous body such as a membrane filter, in order to facilitate preparation and handling of gas-liquid interface culture for inducing differentiation of keratinocytes. A culture plate including a porous body is more preferably used, and a culture plate including a housing portion and a base portion in which the base portion is a porous body is further preferably used as a culture tool. The housing portion is preferably transparent. As the culture plate, a commercially available product may be used. Examples of the commercially available product include a cell culture insert such as Transwell (registered trademark), and the like. The culture plate and the cell culture insert are preferably coated with an appropriate coating agent such as collagen.

The pore size of the membrane filter of a culture tool is not particularly limited as long as the cultured cells can be retained on the membrane filter and the medium component can pass therethrough. For example, the pore size is 0.1 µm to 8 µm, and preferably 0.4 µm to 5 µm. Also, examples of the material of the membrane include polyethylene terephthalate (PET), polycarbonate, polytetrafluoroethylene (PTFE), or the like.

In the method for producing a cultured tissue of the present invention, after an epithelial cell layer is formed, multilayering and cornification of keratinocytes are further promoted to form an epithelial tissue layer. Such a method for inducing multilayering and cornification of keratinocytes is not particularly limited, but can be performed, for example, by performing gas-liquid interface culture to induce differentiation of keratinocytes.

The gas-liquid interface culture is performed by incubating keratinocytes with the surface of the keratinocytes exposed to air after replacing the medium with a cornification medium. The incubation temperature is, for example, 4 to 42°C, preferably 20 to 40°C, and more preferably 30 to 37°C. The incubation time is, for example, 1 day to 40 days, preferably 5 days to 30 days, and more preferably 7 days to 14 days. As the cornification medium (multilayered medium), for example, a medium obtained by adding calcium and/or fetal bovine serum to the medium used for forming the epithelial cell layer or the like can be used. Among them, a medium obtained by mixing a DMEM medium and a Ham's F-12 medium at a ratio of 1 : 1 is suitably used as the cornification medium. The calcium concentration of the cornification medium is preferably, for example, about 0.4 to 2.0 mM.

It is more preferable that one or two or more selected from the group consisting of ethanolamine, O-phosphoethanolamine, hydrocortisone, insulin, adenine, transferrin, selenious acid, triiodothyronine, choline chloride, serine, linoleic acid/BSA, calcium chloride, and ascorbic acid are further added to the cornification medium.

### (Separation step of epithelial tissue layer)

In the method for producing a cultured tissue of the present invention, when the cultured tissue is a cultured epithelium, it is preferable to further include a step of separating the epithelial tissue layer. The operation of separating the epithelial tissue layer is not particularly limited as long as a sheet-like epithelial tissue layer can be obtained, and examples thereof include physical peeling; treatment with a salt such as sodium chloride; treatment with a surfactant such as SDS or Triton-X100; treatment with an enzyme such as dispase or protease; and the like. In the step of separating the epithelial tissue layer, these separation operations can be used singly or in combination of two or more thereof.

### (Other steps)

The method for producing a cultured tissue of the present invention preferably further includes the step of treating fibroblasts with trehalose or a derivative thereof, as a step for preparing fibroblasts treated with a composition containing trehalose or a derivative thereof.

The method for producing a cultured tissue of the present invention may further include a step of preparing a dermal cell structure.

### (Features of production method of present invention)

In the prior art, for example, in order to obtain a cultured epidermis of 20 cm², it is necessary to evenly seed 9 × 10³/cm² to 9 × 10⁴/cm² keratinocytes on a plane, and then culture from proliferation to multilayering and cornification is usually performed for about 21 to 35 days.

When trying to prepare cultured skin with autologous cells without rejection, the number of keratinocytes that can be collected from a living body at one time is limited. Therefore, in the prior art, keratinocytes could not be seeded at the cell density described above without being repeatedly passaged and expanded over several weeks.

According to the method for producing a cultured tissue of the present invention, the step of proliferating keratinocytes can be performed simultaneously during the step of forming an epithelial tissue layer including multilayering and cornification. As a result, the culture time for obtaining a cultured tissue can be greatly shortened. Therefore, the production method of the present invention is suitable for rapidly preparing a self-cultured tissue (for example, self-cultured epithelium, self-cultured skin, self-cultured cornea) of a newly wounded patient.

### (Exemplary specific embodiment)

Hereinafter, one of specific embodiments of the method for producing a cultured tissue of the present invention will be described. These specific embodiments and are merely examples, and the method for producing a cultured tissue of the present invention is not limited thereto.

Here, an embodiment is shown in which fibroblasts treated with trehalose or a derivative thereof are included in a cell support, and a dermal tissue structure and keratinocytes are cultured in a common medium.

The dermal cell structure used in the present embodiment contains at least a cell support and fibroblasts. The shape of the dermal cell structure is not particularly limited, and may take a massive shape, a layered shape, a granular shape, an irregular shape or the like, and among them, a layered substance is preferred.

The fibroblasts may be treated with trehalose or a derivative thereof before or after constituting the dermal cell structure. A specific aspect of the treatment before constituting the dermal cell structure is not particularly limited, but examples thereof include, when proliferating the fibroblast by normal monolayer culture, culturing the fibroblast in a medium containing trehalose or a derivative thereof at the specific concentration described above, and the like. In addition, examples of a specific aspect in which the dermal cell structure is formed and then treated include an aspect in which trehalose or a derivative thereof at the above specific concentration is contained in the cell support, and fibroblasts are treated with trehalose or a derivative thereof while cultured in a medium common to keratinocytes. In this case, the cell support corresponds to the composition containing trehalose or a derivative thereof.

The method for containing trehalose or a derivative thereof at a specific concentration in the cell support in the dermal cell structure is not particularly limited, and examples thereof include the following aspects (i) to (iii). Among them, it is preferable to be treated by the method (i), from the viewpoint of uniformly containing trehalose or a derivative thereof.
(i) Fibroblasts are allowed to coexist in a cell support containing trehalose or a derivative thereof;
(ii) Fibroblasts, a cell support, and trehalose or a derivative thereof are allowed to coexist at a time;
(iii) A structure obtained by containing fibroblasts in a cell support is impregnated with trehalose or a derivative thereof.

The cell support may further contain a medium component such as DMEM, serum such as fetal bovine serum (FCS), ascorbic acid, antibiotics, a growth factor and the like, in order to promote cell growth.

Furthermore, although not particularly limited, it is preferable to include a step of pre-culturing the dermal cell structure under a condition in which keratinocytes are not present. The pre-culture is preferably performed in a medium containing, for example, 10 v/v% FCS, DMEM, and KM, and more preferably performed in a medium containing 10 v/v% FCS, DMEM, KM, and ascorbic acid. The time required for pre-culture is, for example, 1 day to 10 days, more preferably 3 to 7 days, and further preferably 5 days at 37°C.

The medium for pre-culture of the dermal cell structure may further contain, for example, 10 to 300 mg/mL, preferably 20 to 200 mg/mL, more preferably 30 to 150 mg/mL, and further preferably 50 to 100 mg/mL of one or more compounds selected from the group consisting of trehalose and derivatives thereof, in terms of trehalose.

Hereinafter, more specific embodiments of the above specific embodiment will be described.

### <First Embodiment>

As one specific example of the above embodiment, an embodiment in which the cells are cultured in a state where keratinocytes 4 are present on the dermal tissue layer, using a cell culture dish 2 including a cell culture insert 1 as a culture tool and a dermal tissue layer 3 which is a layered structure of a dermal cell structure as a dermal cell structure, is shown in Fig. 1(A). In this specific example, an epithelial tissue layer is formed on the dermal tissue layer, and a three-dimensional cultured tissue can be produced. In addition, a cultured epithelium or a cultured dermis can also be obtained by separating the epithelial tissue layer or the dermal tissue layer from the obtained three-dimensional cultured tissue.

From the viewpoint of strengthening a bond between the epithelial tissue layer and the dermal tissue layer, it is also possible to culture cells by forming a coating film formed of an extracellular matrix component between keratinocytes and the dermal cell structure. As a method for forming such a coating film, for example, a method described in JP-A-2012-205516 can be used.

In the conventional production method not using trehalose or a derivative thereof, contraction of the dermal tissue layer occurs during formation of the epithelial tissue layer on the dermal tissue layer. As a result, peeling of the epithelial tissue layer at the outer edge of the dermal tissue layer may occur, or thickness unevenness, wrinkles, twists or the like may occur in the epithelial tissue layer. However, the production method of the present embodiment has a feature that contraction of the dermal tissue layer does not occur and a sheet-like epithelial tissue layer without wrinkles and twists is formed although mechanism of action is unknown.

### <Second Embodiment>

As one specific example of the above embodiment, there is an embodiment in which the cells are cultured in a state where keratinocytes are present on a plane so as to be separated from the dermal cell structure to form an epithelial tissue layer. An embodiment in which the dermal tissue layer 5 is provided on a bottom surface of a cell culture dish and keratinocytes are cultured on a cell culture insert is shown in Fig. 1(B).

In such an embodiment, since the plane forming the epithelial tissue layer can be covered with a temperature-responsive polymer or the like as described above, it is advantageous for separating the epithelial tissue layer.

### <Third Embodiment>

As one specific example of the above embodiment, there is an embodiment in which the cells are cultured in a state where keratinocytes are present on a plane so as to be separated from the dermal cell structure to form an epithelial tissue layer. An embodiment in which keratinocytes 6 are present on a plane of a bottom surface of a cell culture dish and cultured in a state of being separated from a massive dermal cell structure 7 by a filter insert is shown in Fig. 1(C).

### [Cultured Epithelium, Three-dimensional Cultured Tissue Having Epithelial Tissue Layer and Dermal Tissue Layer, Cultured Dermis]

The cultured epithelium or the three-dimensional cultured tissue according to an embodiment of the present invention is produced by the production method described above. The cultured epithelium or three-dimensional cultured tissue of the present invention has an epithelial tissue layer containing keratinocytes cultured in contact with a common medium together with a dermal cell structure, wherein the dermal cell structure contains trehalose or a derivative thereof, a cell support, and fibroblasts, in which the keratinocytes contain cornified keratinocytes.

The three-dimensional cultured tissue of the present invention further has a dermal tissue layer containing fibroblasts.

The cultured epithelium or three-dimensional cultured tissue of the present invention can be used for evaluation, for example, in a drug efficacy test, a pharmacological test, a safety test and the like of a test substance, in an environment close to actual skin, cornea, or the like. In addition, the cultured epithelium and the three-dimensional cultured tissue of the present invention can also be used as a wound covering graft for treatment of burns and wounds of skin, cornea or the like.

The cultured dermis according to an embodiment of the present invention is obtained by two-dimensionally or three-dimensionally culturing the fibroblasts treated with trehalose or a derivative thereof described in the above section [Method for Producing Cultured Tissue]. Examples of the cultured dermis of the present invention are not particularly limited, but examples thereof include a dermal tissue layer obtained by removing an epithelial tissue layer from the three-dimensional cultured tissue. As a substitute for a dermal tissue such as skin and cornea, with the cultured dermis, for example, evaluation such as a drug efficacy test, a pharmacological test and a safety test of a test substance can be performed in an environment close to actual skin, cornea, or the like. The cultured dermis can also be used as a wound covering graft for treatment of burns and wounds of skin, cornea or the like.

The three-dimensional cultured tissue or the cultured dermis of the present invention can promote angiogenesis and consequently can promote wound healing, as shown in Examples. Therefore, the three-dimensional cultured tissue or the cultured dermis of the present invention is particularly suitably used for, for example, a skin disease caused by blood flow disorder. Examples of such a skin disease include decubitus ulcers, diabetic ulcers, venous stasis ulcers, or arterial ulcers.

The cultured epithelium of the present invention is preferably a cultured skin epithelium or a cultured corneal epithelium, and more preferably a cultured skin epithelium.

The three-dimensional cultured tissue of the present invention is preferably three-dimensional cultured skin or three-dimensional cultured cornea, and more preferably three-dimensional cultured skin.

The cultured dermis of the present invention is preferably a cultured skin dermis or a cultured corneal stroma, and more preferably a cultured skin dermis.

The animal from which the cultured epithelium, three-dimensional cultured tissue and cultured dermis of the present invention are derived is a mammal, and preferably a human.

In the cultured epithelium of the present invention and the three-dimensional cultured tissue of the present invention, for example, tight junctions are preferably formed between adjacent keratinocytes in the epithelial tissue layer. Formation of tight junction in the epithelial tissue layer can be evaluated, for example, by measuring transcutaneous electrical resistance (TER).

In the three-dimensional cultured tissue of the present invention and the cultured dermis of the present invention, the proportion of fibroblasts in lag phase (G2 phase) of cell cycle to the total number of fibroblasts is, for example, 1% or more, preferably 5% or more, more preferably 10% or more, and further preferably 12% or more.

In the three-dimensional cultured tissue of the present invention and the cultured dermis of the present invention, the proportion of fibroblasts in a phase of preparation for mitosis (G2 phase) in cell cycle to the total number of fibroblasts is, for example, 50% or less.

The proportion of cells per cell cycle is determined by flow cytometry (FCM) after separating cells from tissues by a conventional method and staining the cells with a DNA-binding dye (for example, propidium iodide (PI), 7AAD, Hoechst3342, DAPI, and the like) . The presence of G2-phase fibroblasts in the three-dimensional cultured tissue or the cultured dermis is preferable in that proliferation of the epithelial tissue layer can be promoted and bioaffinity of the dermal tissue can be enhanced in applications such as a wound covering graft.

The three-dimensional cultured tissue of the present invention contains Ki67 positive fibroblasts at 5 × 10⁶ to 1 × 10⁸ cells/cm³ and preferably 1 × 10⁷ to 5 × 10⁷ cells/cm³, with respect to the volume of the dermal tissue layer. Ki67 protein is known as a cell proliferative marker. In the present specification, Ki67 positive refers that a cell can be detected by staining with a Ki67 antibody (manufactured by Novocastra Laboratories Ltd) and a secondary antibody after a tissue section is prepared by a conventional method. The fact that the number of Ki67 positive cells is large is preferable in that the bioaffinity of the dermal tissue can be enhanced in applications such as a wound covering graft.

In an embodiment, in the fibroblast contained in the three-dimensional cultured tissue of the present invention or the cultured dermis of the present invention, the mRNA expression level of at least one or more selected from the group consisting of FGF2, EREG, ARG2, CCL2, IL1RN, PGF, SPP1, VEGFA, AREG and DPT is 1.0 times or more or 1.2 times or more, as compared to the mRNA expression level in normal fibroblasts cultured for 3 days in a DMEM medium containing 1% FCS and 1% ABAM in collagen gel embedding. Details of the culture conditions for normal fibroblasts are the conditions described in Examples.

When the three-dimensional cultured tissue or the cultured epithelium of the present invention is used for wound treatment in mammals, it is preferably derived from the same species as the subject to be transplanted, more preferably derived from the same species and the same individual as the subject to be transplanted, and further preferably derived from the subject to be transplanted.

Furthermore, when the three-dimensional cultured tissue or the cultured epithelium of the present invention is used for wound treatment in mammals, it is preferable that the three-dimensional cultured tissue or the cultured epithelium of the present invention does not substantially contain a heterologous component with respect to a target mammal, and it is more preferable that the three-dimensional cultured tissue or the cultured epithelium of the present invention does not contain a component of another individual. Here, the phrase "does not contain a heterologous component or a component of another individual" is not particularly limited, but for example, refers to does not contain a cell or a cell fragment derived from a heterologous or another individual, a secreted protein, an extracellular matrix, serum or the like with respect to a cell constituting a three-dimensional cultured tissue.

The three-dimensional cultured tissue or the cultured epithelium of the present invention is preferably in the form of a sheet from the viewpoint of use in wound treatment of mammals . For example, in the case of cultured skin epithelium, three-dimensional cultured cornea or cultured dermis, the area of the sheet is preferably 50 cm² or more, and more preferably 100 cm² or more. Also, in the case of cultured corneal tissue or three-dimensional cultured cornea, or cultured corneal stroma, the area of the sheet is preferably 1 cm² or more, more preferably 1.5 cm² or more, and further preferably 2 cm² or more.

### [Preparation for External Application]

The preparation for external application according to an embodiment of the present invention contains trehalose or a derivative thereof as an active ingredient, and is used for treatment of an epithelial wound. Sites to be applied are not particularly limited, and examples thereof include skin, cornea, oral cavity, ear, nose, rectum, vagina and the like, but it is preferably skin or cornea, and more preferably skin.

The preparation for external application of the present invention is used for the purpose of preventing, treating, or both of the above diseases, using trehalose or a derivative thereof as a single agent, or using trehalose or a derivative thereof and another drug in combination as a combined agent.

### (Active ingredient: trehalose or derivative thereof)

The preparation for external application of the present invention contains one or two or more compounds selected from the group consisting of trehalose and trehalose derivatives.

The total content of trehalose and a derivative thereof with respect to the total amount of the preparation for external application is, for example, 5% by mass or more, preferably 6% by mass or more, more preferably 8% by mass or more, and further preferably 10% by mass or more, in terms of trehalose, from the viewpoint of remarkably exhibiting the effect of the present invention.

The total content of trehalose and a derivative thereof with respect to the total amount of the preparation for external application is, for example, 80% by mass or less, preferably 70% by mass or less, more preferably 50% by mass or less, and further preferably 30% by mass or less, in terms of trehalose.

The total content of trehalose and a derivative thereof is 5 to 80% by mass, preferably 5 to 30% by mass, more preferably 8 to 30% by mass, and further preferably 10 to 30% by mass, with respect to the total amount of the preparation for external application, from the viewpoint of remarkably exhibiting the effect of the present invention.

The total amount of the preparation for external application when the preparation for external application is a patch is a total weight of a portion of the preparation for external application that can be absorbed into a wound part. That is, it is a total weight of the balance excluding materials containing no active ingredient, such as a support, a liner (release body), a tape and a cloth in the preparation for external application.

In addition, the total amount of the preparation for external application when the preparation for external application is a wound dressing is a total weight of a portion of the preparation for external application that can be absorbed into a wound part at the time of use. That is, it is a total weight of the balance excluding materials containing no active ingredient, such as a support, a liner (release body), a tape and a cloth in a preparation for external application at the time of application to a wound, and it is a weight containing body fluids such as exudate that has permeated into the wound dressing from the wound part, and blood.

### (Base or carrier)

In the preparation for external application of the present invention, the substance as an active ingredient is usually formulated together with a pharmaceutically acceptable base or carrier such as various additives or solvents, and then systemically or locally administered. Here, the pharmaceutically acceptable carrier means a substance other than the active ingredient, which is generally used for pharmaceutical preparation. It is preferable that the pharmaceutically acceptable carrier does not exhibit a pharmacological action at the dosage of the preparation, is harmless, and does not interfere with the therapeutic effect of the active ingredient. The pharmaceutically acceptable carrier can also be used for the purpose of, for example, enhancing usefulness of the active ingredient and the preparation, facilitating the formulation, stabilizing the quality, improving the usability, or the like. Specifically, substances described in "Japanese Pharmaceutical Excipients Directory 2016" published in 2016 by Yakuji Nippo, Limited (edited by IPEC Japan) and the like may be appropriately selected according to the purpose.

The base or carrier used in the preparation for external application of the present invention is not particularly limited, and examples of the oil-soluble base or carrier include petrolatum, purified petrolatum, paraffin, liquid paraffin, lanolin, purified lanolin, hydrocarbons, higher alcohols, vegetable oils, and fatty oils such as animal oils; fatty acid esters, plastibase, glycols, higher fatty acids, and the like. In addition, although not particularly limited, examples of a water-soluble base or carrier include macrogols (polyethylene glycols) such as macrogol 200, macrogol 400, macrogol 1500, macrogol 1540, macrogol 4000, and macrogol 20000; polyhydric alcohols such as concentrated glycerin and propylene glycol; water-soluble polymers such as povidone and polyvinyl alcohol, and the like. Furthermore, the base or carrier to be used in the preparation for external application of the present invention is not particularly limited, and examples thereof include ethanol, isopropyl alcohol, water, glycerin, propylene glycol, polyethylene glycols and the like as water-soluble solvents, liquid oils such as diethyl sebacate, diisopropyl adipate and caprylic/capric triglyceride as oil-soluble solvents, and the like. For the preparation for external application of the present invention, one or two or more of these bases or carriers can be appropriately selected and used.

### (Other components)

The preparation for external application of the present invention may further contain a suspension, a thickener, a stabilizer, a wetting agent, a preservative, an emulsifier, a suspending agent, a pH adjusting agent and the like as necessary.

### (Dosage form)

Examples of dosage form of the preparation for external application of the present invention include skin preparations (e.g., solids for external application, liquids for external application, sprays, ointments, creams, gels, patches, wound dressings, and the like), ophthalmic preparations (e.g., eye drops, eye ointments, and the like), oral preparations (e.g., oral tablets, oral sprays, oral semi-solids, oral rinses, and the like), otic preparations (e.g., ear drops and the like), nasal preparations (e.g., nasal drops and the like), rectal preparations (e.g., suppositories, rectal semi-solids, enema preparations, and the like), vaginal preparations (e.g., vaginal tablets, vaginal suppositories, and the like), and the like. Among them, the dosage form of the preparation for external application of the present invention is preferably an ophthalmic preparation or a skin preparation, more preferably an ointment, a wound dressing, or an eye drop, and further preferably a wound dressing, from the viewpoint of remarkably exhibiting the effect of the present invention. Specific examples thereof will be described below.

### (Dosage form 1: Skin preparation)

### (1) Solid for external application

The solid for external application is a solid preparation applied or scattered on the skin including the scalp or nail, and includes powders for external application and the like. The powder for external application refers to a powdery solid for external application.

### (2) Liquid for external application

The liquid for external application is a liquid preparation applied to the skin including the scalp or nail, and includes liniments, lotions, and the like. The liniment refers to a liquid or muddy liquid for external application used by rubbing against the skin. The lotion refers to a liquid for external application in which an active ingredient is dissolved or emulsified or finely dispersed in an aqueous liquid.

### (3) Spray

The spray is a preparation in which an active ingredient is sprayed onto the skin in the form of mist, powder, foam, paste or the like, and includes aerosols for external application, pump sprays, and the like.

### (4) Ointment

The ointment is a semi-solid preparation in which an active ingredient is dissolved or dispersed in a base, applied to the skin, and includes oil-and-fat ointments, water-soluble ointments, and the like.

### (5) Cream

The cream is a semi-solid preparation emulsified in an oil-in-water type or a water-in-oil type to be applied to the skin, and a lipophilic preparation emulsified in a water-in-oil type may also be referred to as an oily cream.

### (6) Gel

The gel is a gel-like preparation applied to the skin, and includes aqueous gels, oily gels, and the like.

### (7) Patch

The patch is a preparation to be applied to the skin, and includes tapes, cataplasms, and the like. The patch usually refers to a product obtained by using a polymer compound or a mixture thereof as a base, mixing the active ingredient with the base to make the mixture homogeneous, and spreading the mixture on a support or a liner (release body) to mold the mixture. Additives such as a pressure-sensitive adhesive and an absorption promoter can also be used for the patch as necessary. The tape refers to a patch using a base containing almost no water, and includes plaster preparations, plasters, and the like. The tape can be usually produced by using a water-insoluble natural or synthetic polymer compound such as a resin, plastic or rubber as a base, adding the active ingredient as it is, or adding an additive to the active ingredient and adding the mixture to the base, making the whole mixture homogeneous, and spreading the mixture on a cloth or spreading or enclosing the mixture on a plastic film or the like. The cataplasm refers to a patch using a base containing water.

### (8) Wound dressing

The wound dressing is also referred to as dressing and is used to maintain a moist environment of a wound surface in wound treatment. Examples of the wound dressing include a hydrocolloid dressing, a polyurethane foam dressing, an alginate dressing, and the like. At the time of use, a solvent such as water is added, or the body fluid from a wound part comes into contact, whereby a dried active ingredient or the like is converted into a solution and can be delivered to the wound part.

### (Dosage form 2: Ophthalmic preparation)

### (1) Eye drop

The eye drop is applied to ocular tissues such as the cornea, and is a liquid form, or a solid sterile preparation used by dissolving or suspending at the time of use.

### (2) Eye ointment

The eye ointment is a semi-solid sterile preparation to be applied to ocular tissues such as the cornea.

### (Usage)

The preparation for external application of the present invention can be appropriately changed according to the severity of the wound, age, sex and the like of the subject, and for example, an appropriate amount (for example, about 0.05 to 5 g) can be administered (application, eye drop, and the like) to the affected part several times a day (for example, about 1 to 5 times, preferably 1 to 3 times).

The dose per 10 cm² by application of the preparation for external application of the present invention is not particularly limited, but is, for example, 0.1 mg or more, preferably 1 mg or more, more preferably 5 mg or more, further preferably 10 mg or more, and still more preferably 100 mg or more, and 5,000 mg or less, preferably 1,000 mg or less, and more preferably 500 mg or less, in terms of trehalose.

The dose per 10 cm² by application of the preparation for external application of the present invention is not particularly limited, but is, for example, 0.1 to 5,000 mg, preferably 1 to 1, 000 mg, more preferably 5 to 1, 000 mg, further preferably 10 to 500 mg, and still more preferably 100 to 500 mg, in terms of trehalose.

### [Medical Kit]

The medical kit of the present invention includes one or more of the preparations for external applications described above. It is preferable that the medical kit of the present invention includes a suture for wound treatment, a needle, gauze, an adhesive for living body and the like, as articles other than the preparation for external application containing trehalose.

[Composition for ERK1/2 Activation, AKT Activation, FGF2 Expression Promotion, or Cell Proliferation of Fibroblasts]

The composition of the present invention contains trehalose or a derivative thereof, and is used for ERK1/2 activation, AKT activation, FGF2 expression promotion, or cell proliferation of fibroblasts.

### (Trehalose or derivative thereof)

The composition of the present invention contains one or two or more compounds selected from the group consisting of trehalose and trehalose derivatives.

The composition according to an embodiment of the present invention is applied to fibroblasts and activates ERK1/2 that is an MAP kinase. In the present specification, the activation of ERK1/2 refers to an increase in proportion of phosphorylated ERK1/2. A phosphorylation site of ERK1/2 is, for example, Thr202 and Tyr204 in human ERK1/2, and Thr183 and Tyr185 in rat and mouse ERK1/2. It is known that fibroblast proliferation is promoted by the activation of ERK1/2.

The composition according to an embodiment of the present invention is applied to fibroblasts and activates AKT also called protein kinase B, that is a type of serine/threonine kinase. In the present specification, the activation of AKT refers that proportion of AKT in which Ser473 is phosphorylated increases. By the activation of AKT, it is known that the activation of AKT is involved in fibroblast migration, cell proliferation, and survival of fibroblasts in a collagen matrix.

The phosphorylation of ERK1/2 and AKT can be detected and quantified, for example, by Western blotting or ELISA using an antibody specific to each corresponding phosphorylated protein.

The composition according to an embodiment of the present invention is applied to fibroblasts and used to promote expression of FGF2 that is a fibroblast growth factor. In the present specification, the expression promotion of FGF2 refers that the mRNA transcription level from FGF2 gene increases. It is known that increased expression of FGF2 brings about effects of angiogenesis, proliferation of fibroblasts, and promotion of production of hyaluronic acid.

The composition according to an embodiment of the present invention is used to promote expression of EREG, ARG2, CCL2, IL1RN, PGF, SPP1, VEGFA, AREG or DPT in fibroblasts. To promote expression of these genes refers that the mRNA transcription level from these genes is increased.

The composition according to an embodiment of the present invention is applied to fibroblasts, and can also be used for fibroblast proliferation, angiogenesis, or promotion of production of hyaluronic acid.

The composition can also be used for fibroblast activation. Here, the activation of fibroblasts refers to enhancement of function of fibroblasts that promote proliferation of the epithelial tissue layer.

The composition can also be used for improving one or two or more selected from the group consisting of wrinkles, texture, tension, sagging, moisture, resilience, elasticity, and aging of the skin, as the use derived from the above effect on fibroblasts.

The total content of trehalose and a derivative thereof with respect to the total amount of the composition of the present invention is 5% by mass or more, preferably 6% by mass or more, more preferably 8% by mass or more, and further preferably 10% by mass or more, in terms of trehalose, from the viewpoint of remarkably exhibiting the effect of the present invention.

The total content of trehalose and a derivative thereof is, for example, 80% by mass or less, 70% by mass or less, or 50% by mass or less, and preferably 30% by mass or less, with respect to the total amount of the composition of the present invention.

The total content of trehalose and a derivative thereof with respect to the total amount of the composition of the present invention is 5 to 80% by mass, preferably 5 to 30% by mass, more preferably 8 to 30% by mass, and further preferably 10 to 30% by mass, in terms of trehalose, from the viewpoint of remarkably exhibiting the effect of the present invention.

The composition of the present invention is not particularly limited, but may be in the form of, for example, a pharmaceutical product, a quasi-pharmaceutical product, a cosmetic, a food, or the like. In addition, the composition of the present invention can be used, for example, as a culture article, for a cell support such as collagen gel or collagen sponge, a medium, a supplement for adding a medium, serum or the like; a research reagent, or the like.

When the composition of the present invention is a pharmaceutical product, a quasi-pharmaceutical product or a cosmetic, the composition may contain, for example, a base or a carrier, a preservative, an emulsifier, a colorant, an antiseptic, a surfactant, an ultraviolet absorber, an antioxidant, a humectant, an ultraviolet absorber, a fragrance, an antiseptic and antifungal agent, an extender pigment, a coloring pigment, an alcohol, a polyhydric alcohol, water, an oil agent, a thickener, a powder, a chelating agent, an enzyme, animal and plant extracts, a pH adjusting agent, and the like, but not particularly limited thereto.

As a specific example of the base or the carrier, the same one as the base or the carrier in the above section [Preparation for External Application] can be used.

Hereinafter, exemplary embodiments of the present invention will be described.
<1> A method for producing a cultured tissue having an epithelial tissue layer, the production method comprising:
   a step of culturing keratinocytes in contact with a common medium with fibroblasts treated with a composition containing trehalose or a derivative thereof to form an epithelial tissue layer.
<2> The production method according to <1>, wherein
   the fibroblasts are included in a cell support to constitute a dermal cell structure, and
   the dermal cell structure is a layered structure, and the keratinocytes are present on the layered structure of the dermal cell structure.
<3> The production method according to <1> or <2>, further comprising a step of separating the epithelial tissue layer, wherein the cultured tissue having the epithelial tissue layer is a cultured epithelium.
<4> The production method according to any one of <1> to <3>, wherein
   a total content of trehalose and a derivative thereof in the composition containing trehalose or a derivative thereof with respect to the total amount of the composition is 10 mg/mL or more, preferably 20 mg/mL or more, more preferably 30 mg/mL or more, and further preferably 50 mg/mL or more, and
   300 mg/mL or less, preferably 200 mg/mL or less, more preferably 150 mg/mL or less, and further preferably 100 mg/mL or less, in terms of trehalose.
<5> The production method according to any one of <1> to <4>, wherein
   the treatment is carried out by two-dimensional culture of fibroblasts, and the density of fibroblasts in the culture is 3 × 10³ cells/cm² or more, and preferably 5 × 10³ cells/cm² or more, and
   1 × 10⁵ cells/cm² or less, and preferably 8 × 10⁴ cells/cm² or less.
<6> The production method according to any one of <1> to <4>, wherein
   the treatment is carried out by three-dimensional culture of fibroblasts, and the density of the fibroblasts in the culture is 5 × 10³ cells/cm³ or more, preferably 1 × 10⁴ cells/cm³ or more, and more preferably 2 × 10⁴ cells/cm³ or more, and 8 × 10⁵ cells/cm³ or less, preferably 4 × 10⁵ cells/cm³ or less, and more preferably 2 × 10⁵ cells/cm³ or less.
<7> The production method according to any one of <1> to <6>, wherein
   the treatment is carried out at 37°C for 2 hours or more, preferably 5 hours or more, further preferably 12 hours or more, and particularly preferably 24 hours or more, and
   for 10 days or less, 7 days or less, 5 days or less, 3 days or less, or 2 days or less.
<8> The production method according to any one of <1> to <7>, wherein
   the density of the fibroblasts treated with trehalose or a derivative thereof is 5 × 10³ cells/cm³ or more, preferably 1 × 10⁴ cells/cm³ or more, and more preferably 2 × 10⁴ cells/cm³ or more, and
   8 × 10⁵ cells/cm³ or less, preferably 4 × 10⁵ cells/cm³ or less, and more preferably 2 × 10⁵ cells/cm³ or less.
<9> The production method according to any one of <1> to <8>, wherein
   the keratinocytes in whole or in part have a cell density of 2 × 10³ cells/cm² or more, preferably 4.5 × 10³ cells/cm² or more, and more preferably 9 × 10³ cells/cm² or more, and
   3.6 × 10⁵/cm² or less, preferably 1.8 × 10⁵/cm² or less, and more preferably 9 × 10⁴/cm² or less.
<10> The production method according to any one of <1> to <9>, wherein the fibroblasts and the keratinocytes are preferably derived from the same species, more preferably derived from the same species and the same individual as the subject to be transplanted, and further preferably derived from the subject to be transplanted.
<11> The production method according to any one of <2> to <10>, wherein the cell support preferably comprises one or more selected from the group consisting of collagen, gelatin, hyaluronic acid, fibronectin, laminin, fibrin and extracellular matrix mixtures, more preferably comprises collagen, further preferably is a collagen gel or a collagen sponge, and still more preferably is a collagen gel.
<12> The production method according to any one of <2> to <11>, further comprising any one of the following steps (i) to (iii) as a step of preparing the dermal cell structure, and preferably comprising the step (i): (i) allowing fibroblasts to coexist in the cell support containing trehalose or a derivative thereof; (ii) allowing fibroblasts, the cell support, and trehalose or a derivative thereof to coexist at a time; (iii) impregnating a structure obtained by containing fibroblasts in the cell support with trehalose or a derivative thereof.
<13> The production method according to any one of <2> to <12>, wherein the composition containing trehalose or a derivative thereof is the cell support.
<14> A cultured epithelium which is produced by the production method according to any one of <1> to <13>, and is preferably a skin epithelium or a corneal epithelium, and more preferably a human skin epithelium or a human corneal epithelium.
<15> A three-dimensional cultured tissue which is produced by the production method according to any one of <1> to <13>, and has an epithelial tissue layer containing keratinocytes and a dermal tissue layer containing fibroblasts, wherein the proportion of the fibroblasts in G2 phase of cell cycle to the total number of fibroblasts is 1% or more, preferably 5% or more, more preferably 10% or more, further preferably 12% or more, and 50% or less.
<16> The three-dimensional cultured tissue according to <15>, wherein
   Ki67 positive fibroblasts are contained at 5 × 10⁶ cells/cm³ or more and preferably 1 × 10⁷ cells/cm³ or more, and
   1 × 10⁸ cells/cm³ or less and preferably 5 ×10⁷ cells/cm³ or less, with respect to the volume of the dermal tissue layer.
<17> The three-dimensional cultured tissue according to <15> or <16>, wherein the three-dimensional cultured tissue is three-dimensional cultured skin or three-dimensional cultured cornea, preferably human three-dimensional cultured skin or human three-dimensional cultured cornea, and more preferably human three-dimensional cultured skin.
<18> The three-dimensional cultured tissue according to any one of <15> to <17>, wherein the mRNA expression level of at least one or more selected from the group consisting of FGF2, EREG, ARG2, CCL2, IL1RN, PGF, SPP1, VEGFA, AREG and DPT in the fibroblasts is preferably 1.0 times or more and more preferably 1.2 times or more, as compared to the mRNA expression level in normal fibroblasts cultured for 3 days in a DMEM medium containing 1% FCS and 1% ABAM in collagen gel embedding.
<19> The three-dimensional cultured tissue according to any one of <15> to <18>, which is for wound treatment in mammals, and preferably does not contain a heterologous component, and more preferably does not contain a component of another individual.
<20> The three-dimensional cultured tissue according to any one of <15> to <19>, which is a sheet-like three-dimensional cultured skin, wherein the area of the sheet is 50 cm² or more and preferably 100 cm² or more.
<21> The three-dimensional cultured tissue according to any one of <15> to <19>, which is a sheet-like three-dimensional cultured cornea, wherein the area of the sheet is 1 cm² or more, preferably 1.5 cm² or more, and more preferably 2 cm² or more.
<22> A cultured dermis having a dermal tissue layer comprising fibroblasts treated with a composition containing trehalose or a derivative thereof, wherein the proportion of fibroblasts in G2 phase of cell cycle to the total number of fibroblasts is 1% or more, preferably 5% or more, more preferably 10% or more, further preferably 12% or more, and 50% or less.
<23> The cultured dermis according to <22>, wherein
   Ki67 positive fibroblasts are contained at 5 × 10⁶ cells/cm³ or more and preferably 1 × 10⁷ cells/cm³ or more, and
   1 × 10⁸ cells/cm³ or less and preferably 5 × 10⁷ cells/cm³ or less, with respect to the volume of the dermal tissue layer.
<24> The cultured dermis according to <22> or <23>, wherein the cultured dermis is a cultured skin dermis or a cultured corneal stroma, preferably a human cultured skin dermis or a human cultured corneal stroma, and more preferably a human cultured skin dermis.
<25> The cultured dermis according to any one of <22> to <24>, wherein the mRNA expression level of at least one or more selected from the group consisting of FGF2, EREG, ARG2, CCL2, IL1RN, PGF, SPP1, VEGFA, AREG and DPT in the fibroblasts is preferably 1.0 times or more and more preferably 1.2 times or more, as compared to the mRNA expression level in normal fibroblasts cultured for 3 days in a DMEM medium containing 1% FCS and 1% ABAM in collagen gel embedding.
<26> The cultured dermis according to any one of <22> to <25>, which is used for wound treatment in mammals, and preferably does not contain a heterologous component, and more preferably does not contain a component of another individual.
<27> The cultured skin dermis according to any one of <22> to <26>, which is a sheet-like cultured dermis, wherein the area of the sheet is 50 cm² or more, and preferably 100 cm² or more.
<28> The cultured dermis according to any one of <22> to <26>, which is a sheet-like cultured corneal stroma, wherein the area of the sheet is 1 cm² or more, preferably 1.5 cm² or more, and more preferably 2 cm² or more.
<29> A preparation for external application for treatment of an epithelial wound, which comprises trehalose or a derivative thereof as an active ingredient.
<30> The preparation for external application according to <29>, wherein the total content of trehalose and a derivative thereof is 5% by mass or more, preferably 6% by mass or more, more preferably 8% by mass or more, and further preferably 10% by mass or more, and
   80% by mass or less, preferably 70% by mass or less, more preferably 50% by mass or less, and further preferably 30% by mass or less, in terms of trehalose.
<31> The preparation for external application according to <29> or <30>, which further comprises, as a base or a carrier, one or two or more selected from the group consisting of petrolatum, purified petrolatum, paraffin, liquid paraffin, lanolin, purified lanolin, hydrocarbon, higher alcohols, fatty oil, fatty acid esters, plastibase, glycols, higher fatty acid, macrogols, concentrated glycerin, propylene glycol, povidone, polyvinyl alcohol, ethanol, isopropyl alcohol, water, polyethylene glycol, diethyl sebacate, diisopropyl adipate, and caprylic/capric triglyceride.
<32> The preparation for external application according to any one of <29> to <31>, wherein the dosage form is a solid for external application, a liquid for external application, a spray, an ointment, a cream, a gel, a patch, a wound dressing, an eye drop, an eye ointment, an oral tablet, an oral spray, an oral semi-solid, an oral rinse, an ear drop, a nasal drop, a suppository, a rectal semi-solid, an enema preparation, a vaginal tablet, or a vaginal suppository, preferably an ointment, a wound dressing, or an eye drop, and further preferably a wound dressing.
<33> The preparation for external application according to any one of <29> to <32>, which is used for wound treatment of epithelium of skin, cornea, oral cavity, ear, nose, rectum or vagina, preferably for wound treatment of skin or cornea, and more preferably for wound treatment of skin.
<34> Trehalose or a derivative thereof used for wound treatment of epithelium, preferably for wound treatment of epithelium of skin, cornea, oral cavity, ear, nose, rectum or vagina, more preferably for wound treatment of epithelium of skin or cornea, and further preferably for wound treatment of epithelium of skin.
<35> Trehalose or a derivative thereof, in the above <34>, the concentration used is 5% by mass or more, preferably 6% by mass or more, more preferably 8% by mass or more, further preferably 10% by mass or more, and 80% by mass or less, and preferably 70% by mass or less, more preferably 50% by mass or less, and further preferably 30% by mass or less, in terms of trehalose.
<36> A method of treating an epithelial wound, which comprises administering an effective amount of trehalose to a subject in need thereof.
<37> A method, in the above <36>, in which the dose per 10 cm² by application of the preparation for external application is 0.1 mg or more, preferably 1 mg or more, more preferably 5 mg or more, further preferably 10 mg or more, and further preferably 100 mg or more, and
   5, 000 mg or less, preferably 1,000 mg or less, and more preferably 500 mg or less, in terms of trehalose.
<38> Use of trehalose or a derivative thereof for producing a preparation for external application used for treatment of an epithelial wound.
<39> Use of trehalose or a derivative thereof for producing a preparation for external application used for wound treatment of skin epithelium or corneal epithelium.
<40> A medical kit, which comprises the preparation for external application according to any one of <29> to <33>, preferably further comprises one or more selected from the group consisting of a suture for wound treatment, a needle, gauze, and an adhesive for living body.
<41> A composition comprising trehalose or a derivative thereof, which is applied to fibroblasts, and is used for ERK1/2 activation, AKT activation, FGF2 expression promotion, angiogenesis promotion, hyaluronic acid production promotion, cell activation, or cell proliferation.
<42> Use of trehalose or a derivative thereof for producing an agent for ERK1/2 activation, AKT activation, FGF2 expression promotion, an angiogenesis promotion, hyaluronic acid production promotion, cell activation or cell proliferator in fibroblasts.
<43> In the above <41> or <42>, the total content of trehalose or a derivative thereof in the agent or composition is 5% by mass or more, preferably 6% by mass or more, more preferably 8% by mass or more, and further preferably 10% by mass or more, and
   80% by mass or less, preferably 70% by mass or less, more preferably 50% by mass or less, and further preferably 30% by mass or less, in terms of trehalose.
<44> A method for ERK1/2 activation, AKT activation, FGF2 expression promotion, angiogenesis promotion, hyaluronic acid production promotion, cell activation or cell proliferation for fibroblasts, which comprises bringing a composition comprising trehalose or a derivative thereof into contact with fibroblasts.
<45> Use of trehalose or a derivative thereof for ERK1/2 activation, AKT activation, FGF2 expression promotion, angiogenesis promotion, hyaluronic acid production promotion, cell activation or cell proliferation promotion in fibroblasts .

### EXAMPLES

### [Test Example 1: Preparation of Three-Dimensional Cultured Skin Using Trehalose-Containing Dermal Tissue Structure]

### (Method)

### <1. Production of Three-Dimensional Cultured Skin>

(1) First, a gel solution I was prepared with the composition shown in Table 1 below. Subsequently, about 1 mL of the gel obtained was poured into a transwell-collagen coated insert (catalog number: 3492, manufactured by Costar; pore size: 3.0 **µ**m, surface area: 4.67 cm²), which is a cell culture insert, and allowed to stand in an incubator for 5 minutes or more. 5 mL of DMEM containing 10 v/v% FCS and 1 v/v% ABAM (Antibiotics-Antimycotic solution, 100 ×, manufactured by ThermoFisher Scientific Inc.) in which normal human skin fibroblasts (NHDF, manufactured by Cambrex Corporation) were suspended at a cell density of 5 × 10⁵ cells/mL was added to 29 mL of the gel solution I. About 3.5 mL of a gel solution containing the cells was added to the cell culture insert, and the mixture was allowed to stand in a 37°C incubator for 30 minutes to gel.

**[Table 1]**

| Reagent | Blending amount (Parts by mass) |
|---|---|
| 20%FCS/DME/1%ABAM | 50 |
| 8 × DME | 5 |
| Type I collagen | 40 |
| 0.1 M NaOH | 5 |
| Total | 100 |

| | |
|---|---|
| 20% FCS/DME/1% ABAM: obtained by adding 20% (v/v) of FCS and 1% (v/v) of ABAM to DMEM (manufactured by Gibco) 8 × DME: 1.42 g of powdered DMEM + 9.2 ml of milliQ Type I collagen: Cellmatrix (registered trademark), manufactured by Nitta Gelatin Inc. | |

(2) Next, the collagen gel (layered dermal cell structure) containing NHDF was pre-cultured for 5 days using a DMEM medium containing 50 µg/ml ascorbic acid, 10 v/v% FCS, and 1 v/v% ABAM. Next, human normal skin keratinocytes suspended in MCDB medium were seeded on the collagen gel surface so as to be 1.28 × 10⁵ cells/cm², and a medium obtained by mixing equal amounts of MCDB Type-II medium (Growth Medium 2 in JP-B2-3066624) and EGM was added to an external liquid of the cell culture insert and the inside of the insert, followed by culturing at 37°C for 2 days. The composition of EGM is as shown in Table 2.

**[Table 2]**

| Component | Blending amount (mL) |
|---|---|
| DMEM(Gibco) | 375 |
| Ham's F-12 medium | 125 |
| Chelated fatal calf serum | 1.5 |
| 0.1 M Ethanolamine | 0.5 |
| 0.1 M O-Phosphoethanolamine | 0.5 |
| 0.4 mg/mL Hydrocortisone | 0.5 |
| 5 mg/mL Insulin | 0.5 |
| 12.2 mg/mL Adenine | 1 |
| 5 mg/mL Transferrin | 0.5 |
| 6.83 µg/mL Selenious acid | 0.5 |
| 13.46 ng/mL Triiodothyronine | 0.5 |
| 89.3 mg/mL Choline chloride | 0.5 |
| 105.1 mg/mL Serine | 0.5 |
| 20 mg/mL Linoleic acid/BSA | 0.05 |
| 1 M Calcium chloride | 0.4497 |
| 100 × ABAM | 0.5 |
| 50 mg/mL Ascorbic acid | 0.5 |
| 3.14 ng/mL Progesterone | 0.5 |

(3) The medium was replaced with a cornification medium (CM medium), and the cell culture insert was placed such that a porous membrane of the cell culture insert was located at a gas-liquid interface of the cornification medium. In this state, the cells were subjected to gas-liquid interface culture at 37°C for 7 to 14 days to induce differentiation, thereby obtaining three-dimensional cultured skin. The medium was replaced every two days from the start of the culture. The composition of the CM medium is as shown in Table 3.

**[Table 3]**

| Component | Blending amount (mL) |
|---|---|
| DMEM (Gibco) | 250 |
| Ham's F-12 medium | 250 |
| Chelated fatal calf serum | 10 |
| 0.1 M Ethanolamine | 0.5 |
| 0.1 M O-Phosphoethanolamine | 0.5 |
| 0.4 mg/mL Hydrocortisone | 0.5 |
| 5 mg/mL Insulin | 0.5 |
| 12.2 mg/mL Adenine | 1 |
| 5 mg/mL Transferrin | 0.5 |
| 6.83 µg/mL Selenious acid | 0.5 |
| 13.46 ng/mL Triiodothyronine | 0.5 |
| 89.3 mg/mL Choline chloride | 0.5 |
| 105.1 mg/mL Serine | 0.5 |
| 20 mg/mL Linoleic acid/BSA | 0.05 |
| 1 M Calcium chloride | 0.4497 |
| 100 × ABAM | 0.5 |
| 50 mg/mL Ascorbic acid | 0.5 |

### <2. Staining Test>

(4) The obtained tissue sections of each three-dimensional cultured skin were stained with HE, EVG, HABP, K10, K5, Alcian blue, SMA and Ki67 by a conventional method. For Ki67 staining, a Ki67 antibody (manufactured by Novocastra Laboratories Ltd) and a secondary antibody (manufactured by Vector Laboratories) were used.

### <3. Analysis of Cell Proportion per Cell Cycle by Flow Cytometry (FCM)>

(5) Of the obtained three-dimensional cultured skin, a dermal tissue layer composed of fibroblasts was separated and DNA-stained with propidium iodide (PI). Next, the proportion of fibroblasts for each cell cycle was analyzed by FCM using Cycle TEST PLUS DNA Reagent Kit (manufactured by BD).

### (Results)

The obtained three-dimensional cultured skin is shown in Fig. 2. When 10 mg/mL trehalose was added to a collagen gel containing fibroblasts, the area of the epithelial tissue layer formed was slightly enlarged as compared to the case where trehalose was not contained. Furthermore, when 100 mg/mL trehalose was added, the epithelial tissue layer extended to the outer edge of the insert and further expanded to protrude to the outside of the insert.

In addition, in the culture under the conventional condition without trehalose, the collagen gel containing fibroblasts contracts along with the formation of the epithelial tissue layer, and wrinkles or twists may occur in the epithelial tissue layer. However, in the culture to which trehalose was added, contraction of the collagen gel was not observed, and a sheet-like epithelial tissue layer without wrinkles and rumples was obtained.

Furthermore, stained images of the obtained tissue sections of each three-dimensional cultured skin are shown in Fig. 3 and Fig. 4. Except for the stained images of Ki67, no difference in staining pattern depending on the concentration of trehalose was observed. Therefore, it was found that the method of the present invention can obtain three-dimensional cultured skin having collagen, elastic fibers, hyaluronic acid, keratin, mucoid and actin having properties equivalent to those of the conventional method.

On the other hand, in the staining with Ki67 as a cell division marker, as shown in Fig. 4, it was confirmed that the positive cells in the dermal tissue layer increased as the concentration of trehalose increased.

The results of the analysis by FCM are shown in Table 4. It can be seen that the proportions of fibroblasts in S phase (DNA synthesis phase) and G2 phase (a phase of preparation for mitosis) were significantly increased by trehalose. Therefore, it was presumed that the state of fibroblasts was changed in some way by trehalose, and consequently, expansion of the epithelial tissue layer also continued even during gas-liquid interface culture.

**[Table 4]**

| Trehalose concentration (mg/mL) | | 0 | 100 |
|---|---|---|---|
| Proportion of cells in cell cycle (%) | G1 | 97.4 | 62.5 |
| | s | 0.7 | 15.3 |
| | G2 | 0.0 | 14.0 |

| | | | |
|---|---|---|---|
| (n = 2, numerical values are averages) | | | |

On the other hand, when 100 mg/mL trehalose was added to the medium of (2) without collagen gel containing fibroblasts and culture was performed with keratinocytes alone, formation of epithelial tissue layer was not observed as in the absence of trehalose. From these results, it was suggested that the coexistence of the dermal cell structure containing fibroblasts and trehalose brings about an action of promoting the expansion of the epithelial tissue layer.

Furthermore, when 30 mg/mL sucrose or 30 mg/mL fructose was added instead of trehalose to a collagen gel containing fibroblasts and culture was performed in the same manner as described above, no epithelial tissue layer was formed. These sugars were considered to have injured keratinocytes or fibroblasts.

### [Test Example 2: Preparation of Large Scale Three-Dimensional Cultured Tissue]

### (Method)

(1) A layered dermal cell structure containing trehalose at a final concentration of 0 or 100 mg/mL was prepared according to the method (1) of Test Example above except that 10 mL of the gel solution I was added to a cell culture insert with a diameter of 75 mm, and 32 mL of the gel solution containing fibroblasts at the same cell density as in (1) was layered.
(2) A ring with a diameter of 1 cm was placed on the obtained collagen gel, and keratinocytes were seeded inside the ring and cultured in MCDB Type-II-EGM (1: 1) medium for 2 days. As a result, a sheet of keratinocytes (epithelial cell layer) with a diameter of about 1 cm is formed on the collagen gel.
(3) When the keratinocytes became confluent, the ring was removed and the medium was replaced with CM medium, and gas-liquid interface culture was performed for 5 days in the same manner as in the method (3) of Test Example 1.

### (Results)

A photograph of the epithelial tissue layer after gas-liquid interface culture is shown in Fig. 5. In the collagen gel containing no trehalose, the area of the epithelial tissue layer after gas-liquid interface culture did not enlarge from the area at the time of confluence. As described above, in the prior art, it is known that keratinocytes once reaching confluence do not expand even when culture is continued on a wider plane. On the other hand, when a sheet composed of confluent keratinocytes was cultured on a dermal cell structure containing 100 mg/mL trehalose, surprisingly the epithelial tissue layer expanded to the outer edge of the culture insert during gas-liquid interface culture. That is, it was found that when the dermal cell structure in which trehalose was allowed to coexist with fibroblasts and keratinocytes are cultured in a common medium, the keratinocytes can proliferate even after reaching confluence. In addition, it was found that the proliferation rate is sufficiently maintained even when multilayering and cornification has occurred by gas-liquid interface culture.

### [Test Example 3-1. Verification 1 of Effect of Trehalose on Fibroblasts]

### (Method)

(1) NHDF was seeded on a cell culture dish containing DMEM containing 10 v/v% FCS, 1 v/v% ABAM, and trehalose at three concentrations of 0, 10, or 100 mg/mL so as to be 5.5 × 10⁴ cells/cm², and cultured for 24 hours.
(2) The morphology of the cells after culture was observed with an optical microscope. The cells were further DNA-stained with PI and subjected to FCM, and the proportion of each cell cycle was examined.
(3) Furthermore, proteins were extracted from the fibroblasts obtained in (1) using RIPA buffer (1% protein inhibitor (manufactured by SIGMA) + Phosphatase Inhibitor cocktail (manufactured by SIGMA)). Then, 10 µg of protein per sample was separated by SDS-PAGE by a conventional method, transferred to a PVDF membrane, and then subjected to Western blotting. Phosphorylated ERK1/2 and phosphorylated AKT were detected by ECL Prime Western Blotting Detection Reagent (manufactured by GE Healthcare), using Phospho-p44/p42 MAPK (Erk1/2) (Thr202/Tyr204) (D13.14.4E)XP Rabbit mAb (#4300, Cell Signaling) and Phospho-Akt(Ser473) antibody (#9271, manufactured by Cell Signaling Technology) as a primary antibody, and an anti-rabbit IgG antibody and an anti-mouse IgG antibody manufactured by PROMEGA were used as secondary antibodies, and visualized by Image Quant LAS4010 (manufactured by GE Healthcare).

### (Results)

Light microscopic images of fibroblasts cultured in a monolayer at each trehalose concentration are shown in Fig. 6. In the absence of trehalose, elongated cells were arranged almost without gaps. This was similar to cell morphology usually seen when cultured to sub-confluence. On the other hand, when 10 mg/mL trehalose was added, the increase in the number of cells was slightly suppressed, and as a result, gaps were observed between the cells. Further, when 100 mg/mL trehalose was added, the increase in the number of cells was further suppressed, but there was no appearance of any damage. As for the cell morphology, there was a tendency that the number of cells extending long cell protrusions was slightly small and the number of spindle-shaped cells was large.

In addition, according to the results of Western blotting (Fig. 7), it was found that phosphorylation of ERK1/2 and AKT is promoted as the concentration of trehalose in the medium increases. In addition, the synthesis amount of β-actin also increased depending on the amount of trehalose.

### [Test Example 3-2. Verification 2 of Effect of Trehalose on Fibroblasts]

### (Method)

(1) NHDF was seeded on a cell culture dish containing 1% FCS and 1% ABAM-containing DMEM medium containing 0, 30, and 100 mg/mL trehalose so as to be 5.5 × 10⁴ cells/cm², and cultured for 3, 24, or 48 hours.
(2) Cells under each condition were collected, and mRNA was extracted using RNeasy Mini Kit (manufactured by QIAGEN) . cDNA was prepared from mRNA using iScript cDNA Synthesis kit (manufactured by B10-RAD Laboratories). The method was performed according to each manual. Next, quantitative PCR (qPCR) of FGF2 gene was performed. For the reaction, Fast Start Universal Probe Master (Rox) (Roche) and StepOnePlue Real time PCR system were used. As a primer set, TaqMan probes for FGF2 (ThermoFisher Scientific) described in Table 5 were used. GAPDH was used as a reference gene. The number of samples used in each condition was 3.

**[Table 5]**

| Gene | TaqMan Probe Assay ID | Gene | TaqMan Probe Assay ID |
|---|---|---|---|
| FGF2 | Hs00960934_m1 | VEGFA | Hs00900055 _m1 |
| EREG | Hs00914313_m1 | DPT | Hs00355056 _m1 |
| AREG | Hs00950669_m1 | CCL2 | Hs00234140 _m1 |
| GAPDH | Hs02758991_g1 | SPP1 | Hs00959010_m1 |
| ARG2 | Hs00982833_m1 | IL1RN | Hs00893626_m1 |
| PGF | Hs00182176 _m1 | | |

### (Results)

The results of qPCR are shown in Fig. 8. After 3 hours of culture, an increase in FGF2 expression level was observed in the fibroblasts treated with 30 mg/mL or 100 mg/mL trehalose as compared to the control without trehalose (vehicle) . In the control, the expression level of FGF2 mRNA after a lapse of 48 hours from culture was significantly reduced as compared to that at the start of the culture. When treated with 30 mg/mL trehalose, the expression level of FGF2 mRNA after 48 hours was slightly increased. Furthermore, when treated with 100 mg/mL trehalose, the FGF2 mRNA expression levels after 24 hours and 48 hours were significantly increased as compared to the control, and had a remarkable feature that the FGF2 mRNA expression levels were still also increased as compared to that at the start of the culture. Therefore, the FGF2 gene can be used as a marker gene for determining whether or not the fibroblast has been subjected to trehalose treatment.

From the above, it is presumed that culturing in the presence of trehalose changes the expression of genes in fibroblasts, and as a result, the culturing improves the effect of proliferating the epithelial tissue layer by the fibroblast.

### [Test Example 3-3. Verification 3 of Effect of Trehalose on Fibroblasts]

In the same manner as in Test Example 3-2, NHDF was cultured in 1% FCS and 1% ABAM-containing DMEM medium containing 0, 30, or 100 mg/mL trehalose or hyaluronic acid oligosaccharide 4mer (HA4) (product code 11006, manufactured by Cosmo Bio Co., Ltd.) for 24 hours, and RNA was recovered from the cells . Also, as a control, RNA was recovered from fibroblasts before treatment (0 h in Fig. 9). The obtained sample was subjected to the same operation as in Test Example 3-2, and qPCR for genes EREG, ARG2, CCL2, IL1RN, PGF, SPP1, VEGFA, AREG and DPT was performed. GAPDH was used as a reference gene.

The results were shown in Fig. 9. In the group cultured in a medium containing 100 mg/mL trehalose (Treha 100), mRNA expression levels of genes EREG, ARG2, CCL2, IL1RN, PGF, SPP1, VEGFA, AREG and DPT in fibroblasts were remarkably increased as compared to those in the group cultured in a medium containing no trehalose (vehicle) . On the other hand, in the group treated with hyaluronic acid oligosaccharides (HA oligo), such an increase in expression level was not observed.

### [Test Example 3-4. Verification 4 of Effect of Trehalose on Fibroblasts]

A collagen gel containing NHDF was prepared according to the method (1) of Test Example 1. The obtained collagen gel was cultured in DMEM medium containing 0 or 100 mg/mL trehalose, 1% FCS and 1% ABAM for 3 days, and RNA was recovered from the cells. The obtained sample was subjected to the same operation as in Test Example 3-2, and qPCR for genes EREG, ARG2, CCL2, IL1RN, PGF, SPP1, VEGFA, DPT and FGF2 was performed. GAPDH was used as a reference gene.

The results were shown in Fig. 10. In the group cultured in a medium containing 100 mg/mL trehalose (Treha 100), as in Test Example 3-3, mRNA expression levels of genes EREG, ARG2, CCL2, IL1RN, PGF, SPP1, VEGFA, DPT and FGF2 in fibroblasts significantly increased as compared to the group cultured in a trehalose-free medium (vehicle) . Therefore, these genes can be used as marker genes for determining whether or not the fibroblasts have been subjected to trehalose treatment.

### [Test Example 4. Preparation of Three-Dimensional Cultured Skin Using Fibroblasts Pretreated with Trehalose]

### (Method)

(1) NHDF was seeded on a cell culture dish containing 10% FCS and 1% ABAM-containing DMEM medium at a cell density of 4.0 × 10⁴ cells/cm². After 24 hours from seeding, at a cell density of 4.5 × 10⁴ cells/cm², the medium was replaced with 1% FCS and 1% ABAM-containing DMEM medium containing 0, 30, or 100 mg/mL trehalose, and the cells were incubated at 37°C for 24 hours. Then, a collagen gel containing 5.0 × 10⁵ cells/ml of the obtained NHDF was prepared under the same conditions as in Test Example 1 except that trehalose was not added.
(2) According to the method of Test Example 1, the obtained NHDF-containing collagen gel was pre-cultured for 5 days, and then human normal skin keratinocytes were seeded and cultured for 2 days at 37°C.
(3) Further, according to the method of Test Example 1, gas-liquid interface culture was performed at 37°C for 7 days using CM medium.

### (Results)

The obtained three-dimensional cultured skin is shown in Fig. 11. When fibroblasts pre-cultured with 30 mg/mL trehalose before preparation of a collagen gel were used, the area of three-dimensional cultured skin was enlarged (about 1.4 times in diameter) as compared to the case of pre-culturing with a medium containing no trehalose. Then, when fibroblasts pre-cultured with 100 mg/mL trehalose were used, the area of three-dimensional cultured skin was enlarged to cover the entire cell culture insert. In addition, in the conventional culture, contraction of the collagen gel may be observed after multilayering, but when fibroblast cells pre-cultured with trehalose were used, contraction of the collagen gel was not observed. This result was surprisingly very similar to the result obtained in Test Example 1. Therefore, it was found that the fibroblasts once treated with trehalose exhibit an effect of suppressing contact inhibition of keratinocytes and expanding the epithelial tissue layer even in the absence of trehalose in the subsequent culture.

### [Test Example 5. Verification of Effect by In Vivo Transplantation of Three-Dimensional Cultured Skin (Skin Sheet)]

### (Method)

(1) Under 5% isoflurane inhalation anesthesia, skin ulcers of about 6 mm in diameter were prepared on the back of BALB/cAJcl-nu nude mice (2 groups of 1 male and 1 female mice of 10 weeks of age in each group) using trepan of 6 mm size.
(2) A skin sheet obtained by the production method of the present invention and a skin sheet prepared by a conventional method not using trehalose were prepared.
(3) These two types of skin sheets were transplanted into the ulcer part of each group of nude mice, and fixed with Decaderm^{™} (manufactured by 3M).
(4) A skin biopsy of the transplantation part was performed 5 days after the transplantation. HE was used for staining.

### (Results)

Tissue staining images of the transplantation parts are shown in Fig. 12. It was found that the trehalose group transplanted with the skin sheet prepared by the production method of the present invention had a significantly larger number of new blood vessels than the control group transplanted with the skin sheet prepared by the production method of the prior art.

### [Test Example 6. In Vivo Test of Effect of Trehalose on Skin Wound]

### (Method)

In order to verify an effect of trehalose on skin wounds, in vivo test using mice was performed. The method is shown below.
(1) 15 week old C57BL/6 mice were anesthetized, the back was extensively depilated, then the skin of the back was pinched and overlaid, and a wound with a diameter of 6 mm was prepared with a biopsy trepan.
(2) Wound mice were divided into 2 groups of 6 mice each, and 100 mg of an agent composed only of Vanicream (Pharmaceutical Specialties, INC) as a base was applied to a control group, and 100 mg of an agent composed of 10% by mass trehalose and Vanicream as a base was applied to a trehalose treatment group once a day.
(3) Images of each wound up to 9 days after wound formation and the area of each wound were recorded, respectively.

### (Results)

The results are shown in Fig. 13. It can be seen that recovery of wounds of the trehalose treatment group is significantly faster than that of the control group. In the trehalose treatment group, regeneration of the skin from the edge of the wound was observed, and wound area was remarkably reduced after only 1 day and epithelialized on day 4.

From the above, it was found that trehalose promotes improvement of an epithelial wound of a living body and is suitable for treatment. When considered in conjunction with Test Examples 1 to 5, it is presumed that, by trehalose, activation of fibroblasts and proliferation of keratinocytes at the edge of the wound were promoted, and regeneration of the skin was promoted.

### INDUSTRIAL APPLICABILITY

The present invention includes an artificial skin model applicable to wound treatment and safety evaluation of a composition for external application, and a preparation for external application for wound treatment. The present invention also includes uses of trehalose such as fibroblast gene activation. Therefore, the present invention is useful, for example, in the fields of cosmetics, medicine, pharmaceuticals, research reagents, foods, and the like.

## Claims

1. A method for producing a cultured tissue having an epithelial tissue layer, the production method comprising:
a step of culturing keratinocytes in contact with a common medium with fibroblasts treated with a composition containing trehalose or a derivative thereof to form an epithelial tissue layer.

2. The production method according to claim 1, wherein
the fibroblasts are included in a cell support to constitute a dermal cell structure, and
the dermal cell structure is a layered structure, and the keratinocytes are present on the layered structure of the dermal cell structure.

3. The production method according to claim 1 or 2, further comprising a step of separating the epithelial tissue layer,
wherein the cultured tissue having the epithelial tissue layer is a cultured epithelium.

4. The production method according to any one of claims 1 to 3, wherein a total content of trehalose and a derivative thereof in the composition containing trehalose or a derivative thereof is 10 to 300 mg/mL in terms of trehalose.

5. A cultured tissue comprising keratinocytes cultured in contact with a common medium together with a dermal cell structure,
wherein the dermal cell structure comprises trehalose or a derivative thereof, a cell support, and fibroblasts,
wherein the keratinocytes contain cornified keratinocytes.

6. A cultured tissue having a dermal tissue layer comprising at least fibroblasts, wherein the proportion of the fibroblasts in G2 phase of cell cycle to a total number of the fibroblasts is 1 to 50%.

7. A preparation for external application for treatment of an epithelial wound, comprising trehalose or a derivative thereof as an active ingredient.

8. The preparation for external application according to claim 7, wherein a total content of trehalose and a derivative thereof is 5 to 30% by mass in terms of trehalose.

9. The preparation for external application according to claim 7 or 8, which is a skin preparation, an ophthalmic preparation, an oral preparation, an otic preparation, a nasal preparation, a rectal preparation, or a vaginal preparation.

10. A composition comprising trehalose or a derivative thereof, which is applied to fibroblasts, and is used for ERK1/2 activation, AKT activation, FGF2 expression promotion, cell activation, or cell proliferation.
